(19) Europäisches Patentamt
European Patent Office
Office européen des brevets

(11) EP 4 270 574 A1

(12) **EUROPEAN PATENT APPLICATION**
published in accordance with Art. 153(4) EPC

(43) Date of publication:
**01.11.2023 Bulletin 2023/44**

(21) Application number: **21913932.6**

(22) Date of filing: **16.12.2021**

(51) International Patent Classification (IPC):
***H01M 10/0525*** *(2010.01)* ***H01M 10/0567*** *(2010.01)*
***H01M 10/0566*** *(2010.01)* ***H01M 4/485*** *(2010.01)*
***H01M 4/505*** *(2010.01)* ***H01M 4/525*** *(2010.01)*

(52) Cooperative Patent Classification (CPC):
**H01M 4/485; H01M 4/505; H01M 4/525; H01M 10/0525; H01M 10/0566; H01M 10/0567; H01M 10/0568; H01M 10/0569;** Y02E 60/10

(86) International application number:
**PCT/CN2021/138677**

(87) International publication number:
**WO 2022/143191 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2020 CN 202011606717**
**13.04.2021 CN 202110392954**

(71) Applicant: **Shenzhen Capchem Technology Co., Ltd**
**Shenzhen, Guangdong 518118 (CN)**

(72) Inventors:
- **HU, Shiguang**
  **Shenzhen, Guangdong 518118 (CN)**
- **QIAN, Yunxian**
  **Shenzhen, Guangdong 518118 (CN)**
- **CAO, Chaowei**
  **Shenzhen, Guangdong 518118 (CN)**
- **GUO, Pengkai**
  **Shenzhen, Guangdong 518118 (CN)**
- **XIANG, Xiaoxia**
  **Shenzhen, Guangdong 518118 (CN)**
- **WANG, Chi**
  **Shenzhen, Guangdong 518118 (CN)**
- **XIANG, Shuhuai**
  **Shenzhen, Guangdong 518118 (CN)**
- **CHEN, Qun**
  **Shenzhen, Guangdong 518118 (CN)**
- **DENG, Yonghong**
  **Shenzhen, Guangdong 518118 (CN)**

(74) Representative: **Maiwald GmbH**
**Elisenhof**
**Elisenstraße 3**
**80335 München (DE)**

# (54) LITHIUM-ION BATTERY

(57) In order to overcome the problems of serious aerogenesis and poor high-temperature cycling performance of existing high-nickel and high-voltage lithium-ion batteries, provided in the present invention is a lithium-ion battery, comprising a positive electrode, a negative electrode, and a non-aqueous electrolyte. The positive electrode comprises a positive electrode material layer, and the positive electrode material layer comprises a positive electrode active material, comprising $LiNi_xCo_yMn_zL_{(1-x-y-z)}O_2$, wherein L is Al, Sr, Mg, Ti, Ca, Zr, Zn, Si, Cu, V or Fe, 0.5≤x≤1, 0≤y≤0.5, 0≤z≤0.5, 0≤x+y+z≤1, and the upper limit voltage of the lithium-ion battery is greater than or equal to 4.2 V; and the non-aqueous electrolyte comprises a solvent, an electrolyte salt, and a compound as represented by structural formula 1: A-D-B-E-C, structural formula 1. On the basis of the total mass of the non-aqueous electrolyte being 100%, the added amount of the compound represented by the structural formula 1 is 0.01 to 5.0%. By means of the present invention, the compound represented by the structural formula 1 is used in combination with a high-nickel ternary material to obtain a ternary high-nickel lithium-ion battery with high high-temperature circulating capacity retention rate and less circulating aerogenesis.

EP 4 270 574 A1

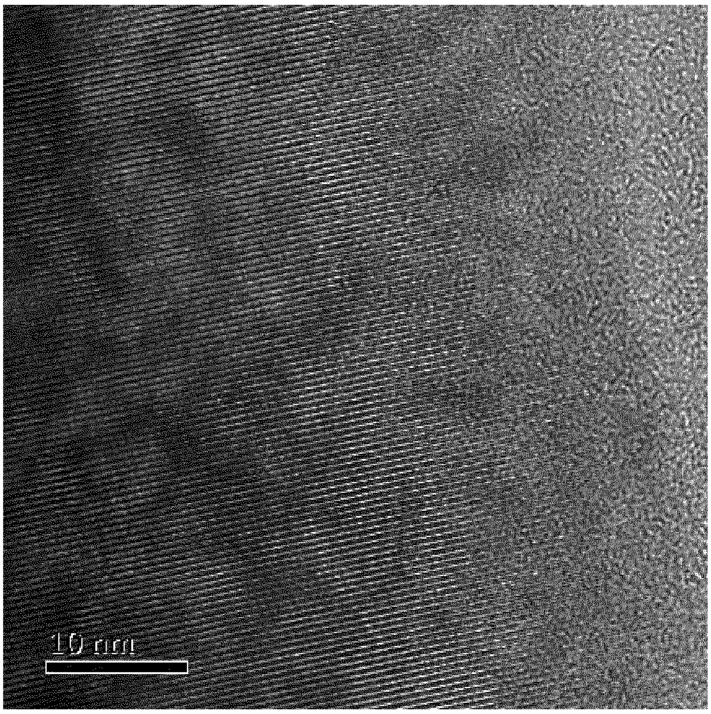


FIG. 1

## Description

### TECHNICAL FIELD

The present disclosure generally relates to a technical field of secondary batteries, and more particularly to a lithium-ion battery.

### BACKGROUND

A lithium-ion battery has been widely used in life and production due to its excellent performance. In recent years, with the development of consumer electronics and new energy vehicles, people have put forward higher requirements for the performance of the lithium-ion battery, especially the cycling performance needs to be further improved under high-temperature conditions. In a cycling process of the lithium-ion battery, especially a high-nickel and high-voltage ternary battery system, when the nickel content and working voltage in the ternary positive electrode material are relatively high, the gas production of the lithium-ion battery during the cycling is more serious. The possible reasons are as follows: on the one hand, with the increase in the nickel content, the original content of alkaline compounds on the surface of the positive electrode material increases, especially the content of lithium carbonate. During the cycling of the battery, lithium carbonate will decompose to generate gas. On the other hand, the proportion of lithium ions that can be deintercalated in the high-nickel ternary material is larger, and the structure of the positive electrode material is easily changed or even collapsed at this time, which will cause the rupture of the positive electrode protective film and cause a large amount of gas to be generated by the side reactions when the positive electrode material is directly exposed to an electrolyte. At the same time, the activity of nickel ions is high, and it is easier and faster for the electrolyte to oxidize and decompose on the surface of the positive electrode material with high nickel content, which further increases the gas production during the cycling and deteriorates the high-temperature cycling performance.

### SUMMARY

With regard to the problems of severe gas production and poor high-temperature cycling performance of the existing lithium-ion battery, the present disclosure provides a lithium-ion battery.

The technical solution used in the present disclosure to solve the above-mentioned technical problem is as follows:

The present disclosure provides a lithium-ion battery, including a positive electrode, a negative electrode and a non-aqueous electrolyte. The positive electrode includes a positive electrode material layer, the positive electrode material layer includes a positive electrode active material, and the positive electrode active material includes $LiNi_xCo_yMn_zL_{(1-x-y-z)}O_2$, where L is Al, Sr, Mg, Ti, Ca, Zr, Zn, Si, Cu, V or Fe, $0.5 \leq x \leq 1$, $0 \leq y \leq 0.5$, $0 \leq z \leq 0.5$, $0 \leq x + y + z \leq 1$, and an upper limit voltage of the lithium-ion battery is $\geq 4.2$ V. The non-aqueous electrolyte includes a solvent, an electrolyte salt and a compound represented by formula 1:

A-D-B-E-C      Formula 1

- where A, B, and C are each independently selected from a group containing a cyclic carbonate group, a cyclic sulfate group, a cyclic sulfite group, a cyclic sulfonate group, a cyclic sulfone group, a cyclic sulfoxide group, a cyclic carboxylate group or a cyclic anhydride group;
- D and E are each independently selected from a single bond, or a group containing a hydrocarbylene group, an ether bond, a sulfur-oxygen double bond or a carbon-oxygen double bond.

Based on a total mass of the non-aqueous electrolyte as 100%, the compound represented by the formula 1 is added in an amount of 0.01 to 5.0%.

Optionally, A, B, and C each independently contain 1 to 5 of the cyclic carbonate group(s), the cyclic sulfate group(s), the cyclic sulfite group(s), the cyclic sulfonate group(s), the cyclic sulfone group(s), the cyclic sulfoxide group(s), the cyclic carboxylate group(s) or the cyclic anhydride group(s), and a total number of the cyclic carbonate group(s), the cyclic sulfate group(s), the cyclic sulfite group(s), the cyclic sulfonate group(s), the cyclic sulfone group(s), the cyclic sulfoxide group(s), the cyclic carboxylate group(s) or the cyclic anhydride group(s) of A, B, and C is less than or equal to 10.

Optionally, A and C are each independently selected from a group represented by formula 2:

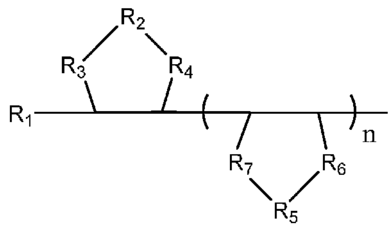

Formula 2

where n is selected from an integer of 0 to 4, and $R_1$ is selected from hydrogen, halogen or a C1 to C5 halohydrocarbonyl group; $R_2$, $R_3$, $R_4$, $R_5$, $R_6$, and $R_7$ are each independently selected from a C1 to C3 hydrocarbylene group, a C1 to C3 alkoxy group, an oxygen atom,

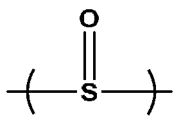,

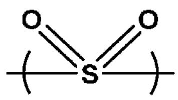, or 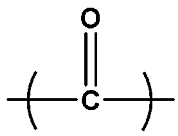,

in which at least one of $R_2$, $R_3$, and $R_4$ is selected from

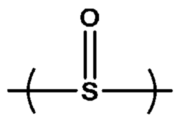, 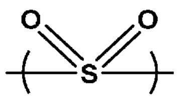, or 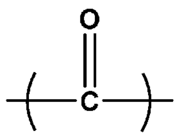,

and at least one of $R_2$, $R_3$, and $R_4$ is selected from the oxygen atom, and at least one of $R_5$, $R_6$, and $R_7$ is selected from

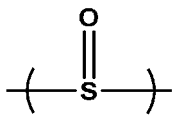, 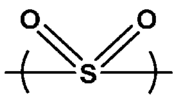,

or

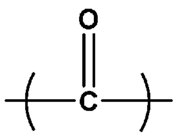,

and at least one of $R_5$, $R_6$, and $R_7$ is selected from the oxygen atom.

Optionally, B is selected from a group represented by formula 3:

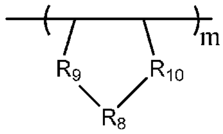

Formula 3

where m is selected from an integer of 1 to 4, and $R_8$, $R_9$, and $R_{10}$ are each independently selected from a C1 to C3 hydrocarbylene group, a Cl to C3 alkoxy group, an oxygen atom,

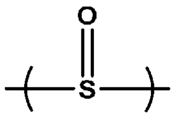, 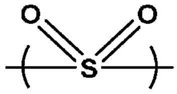, or 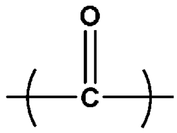,

at least one of $R_8$, $R_9$, and $R_{10}$ is selected from

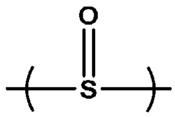, 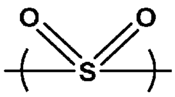, or 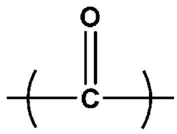,

and at least one of $R_8$, $R_9$, and $R_{10}$ is selected from the oxygen atom.

Optionally, D and E are each independently selected from a group represented by formula 4:

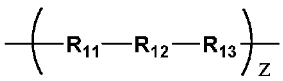

Formula 4

where z is selected from an integer of 0 to 4, $R_{11}$ and $R_{13}$ are each independently selected from a single bond or a C1 to C5 hydrocarbylene group, and $R_{12}$ is selected from a single bond,

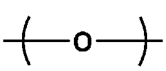, 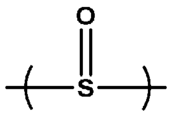, 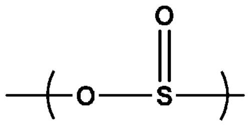, 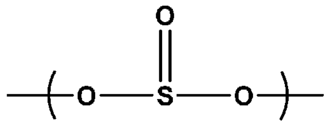, 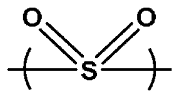,

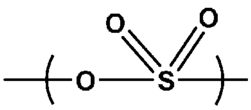, 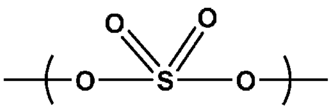, 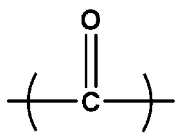, 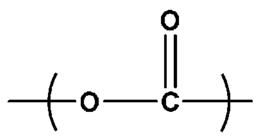

or

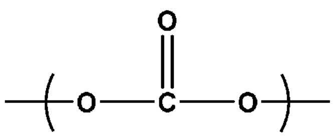.

Optionally, D and E are each independently selected from the single bond or the C1 to C5 hydrocarbylene group, and A, B, and C are each independently selected from the cyclic carbonate group, the cyclic sulfate group, the cyclic sulfite group, the cyclic sulfonate group, the cyclic sulfone group, the cyclic sulfoxide group, the cyclic carboxylate group or the cyclic anhydride group, which is substituted or unsubstituted.

Optionally, when A, B or C is substituted, a substituent is selected from halogen, a hydrocarbonyl group or a halohydrocarbonyl group.

Optionally, when A, B or C is substituted, the substituent is selected from halogen, an alkyl group or a haloalkyl group.

Optionally, A and C are same as each other, A and B are same as or different from each other, and D and E are same as each other.

Optionally, the compound represented by the formula 1 is selected from one or more of following compounds:

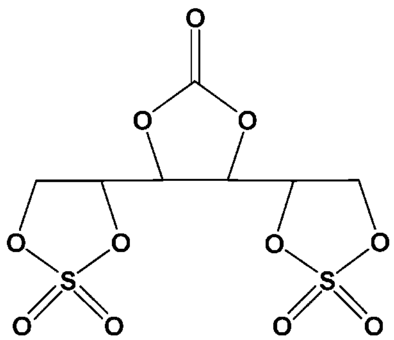

Compound 1

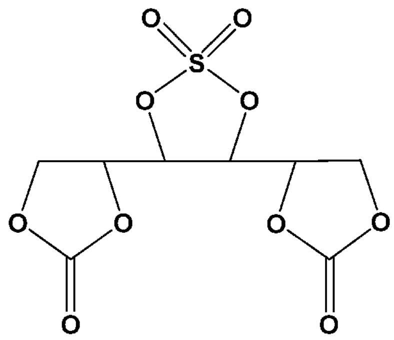

Compound 2

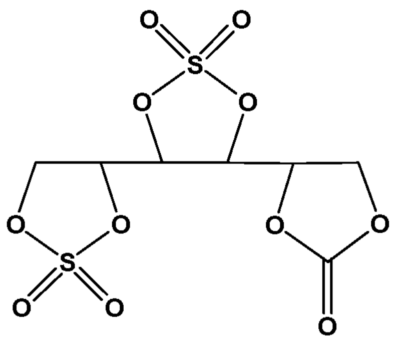

Compound 3

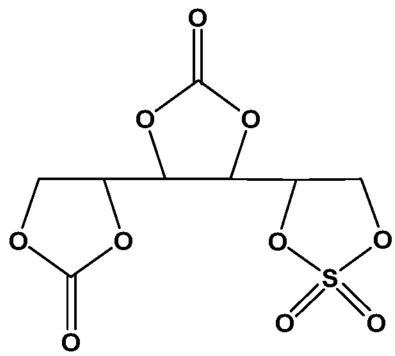

Compound 4

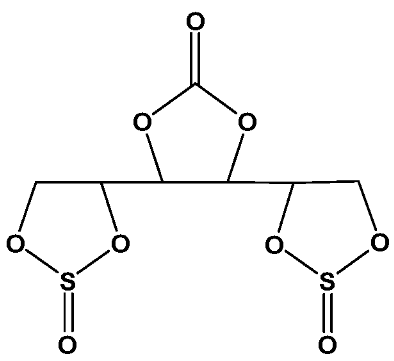

Compound 5

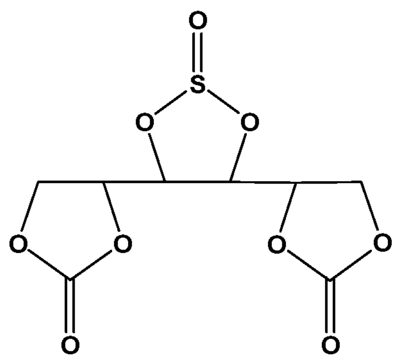

Compound 6

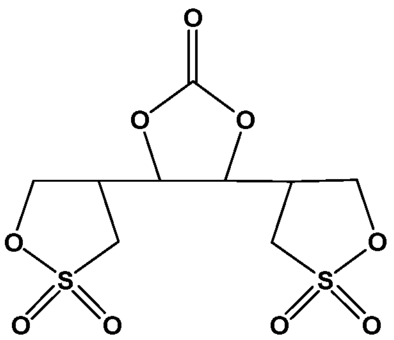

Compound 7

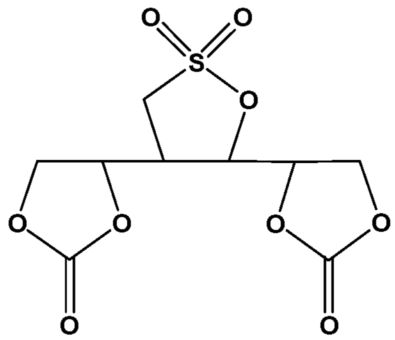

Compound 8

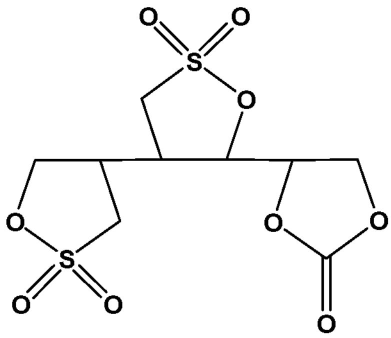

Compound 9

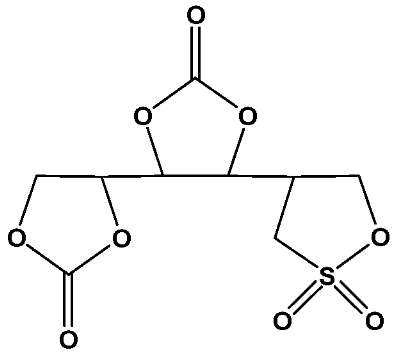

Compound 10

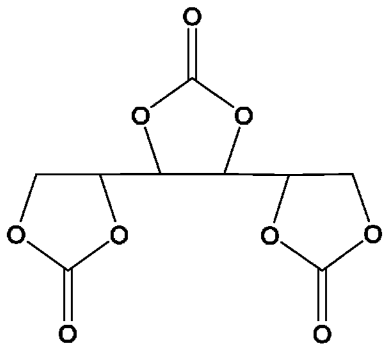

Compound 11

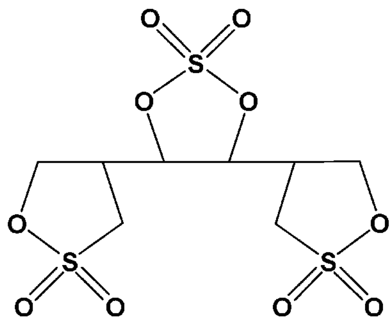

Compound 12

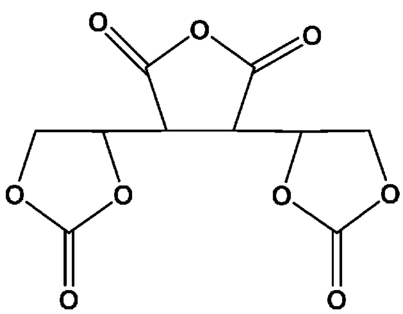

Compound 1-13

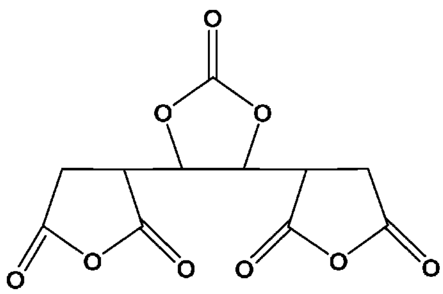

Compound 1-14

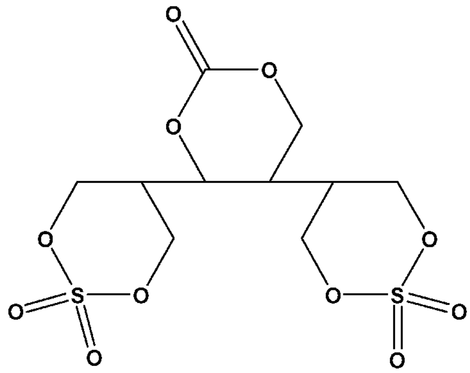

Compound 1-15

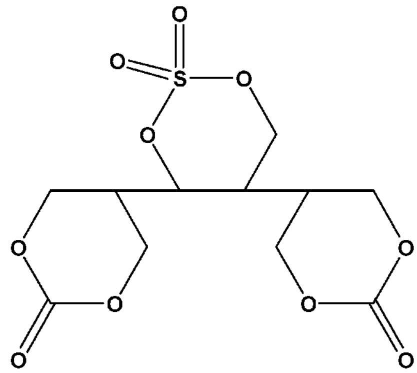

Compound 1-16

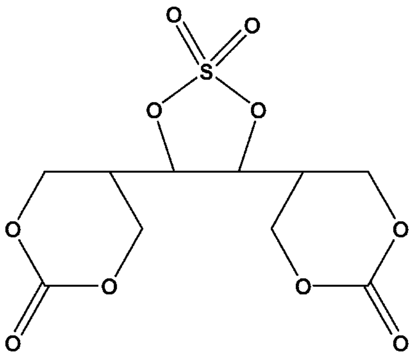

Compound 17

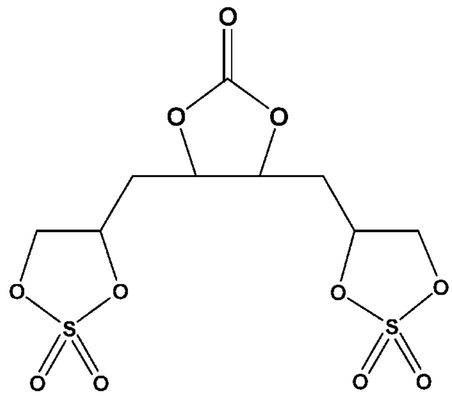

Compound 18

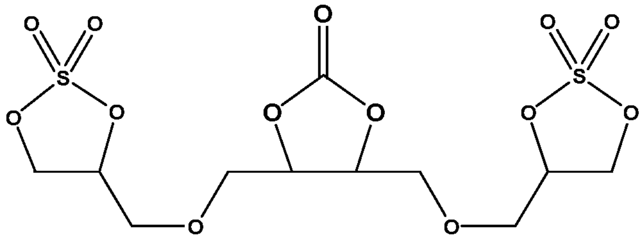

Compound 19

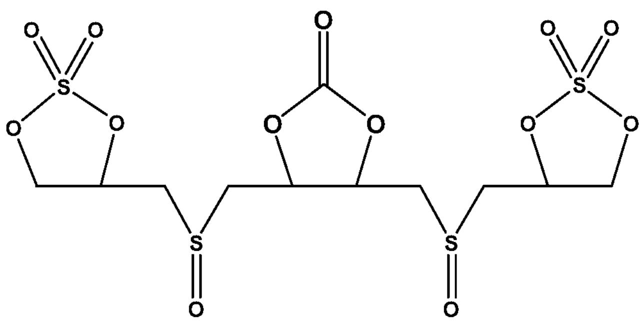

Compound 20

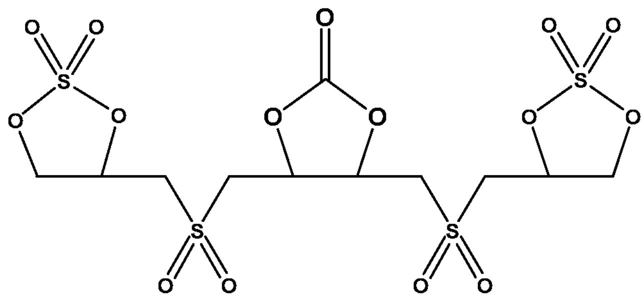

Compound 21

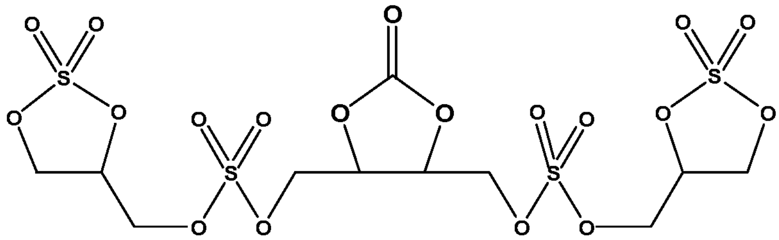

Compound 22

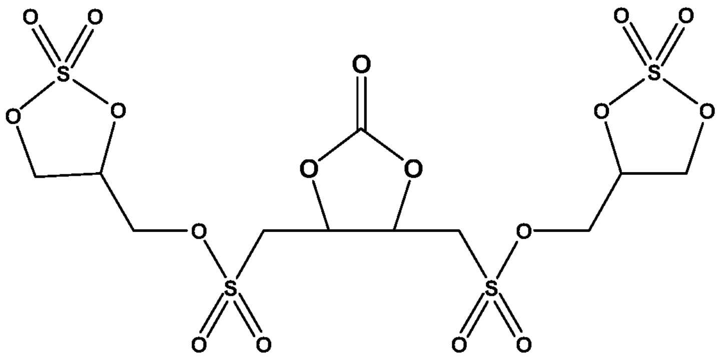

Compound 23

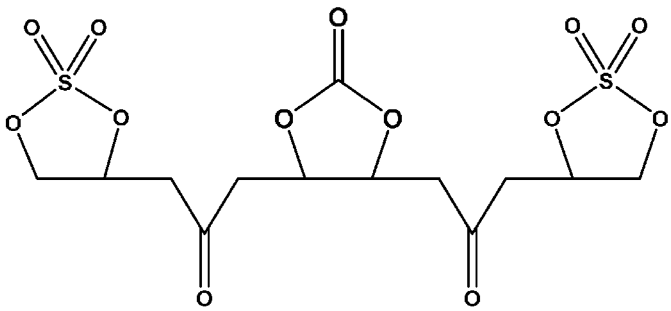

Compound 24

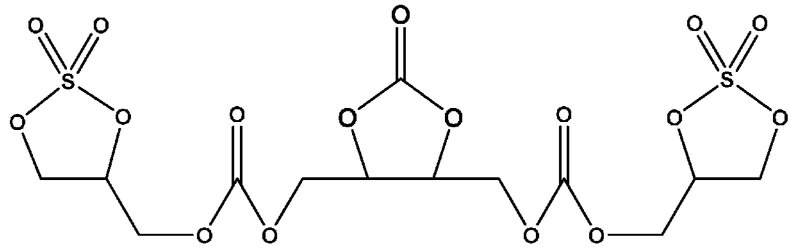

Compound 25

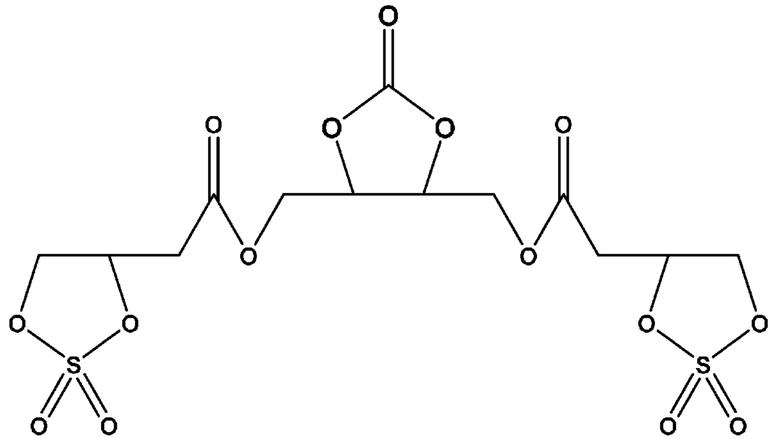

Compound 26

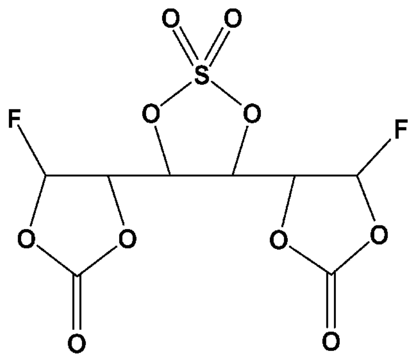

Compound 27

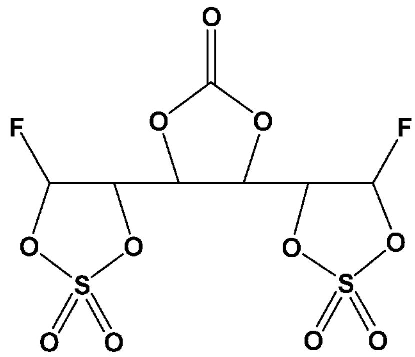

Compound 28

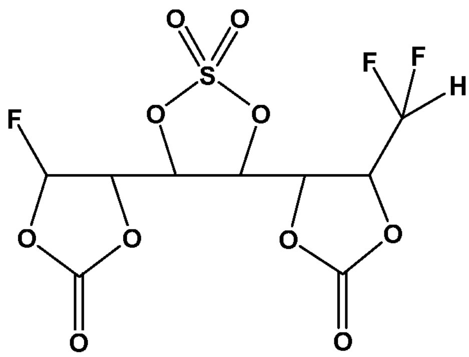

Compound 29

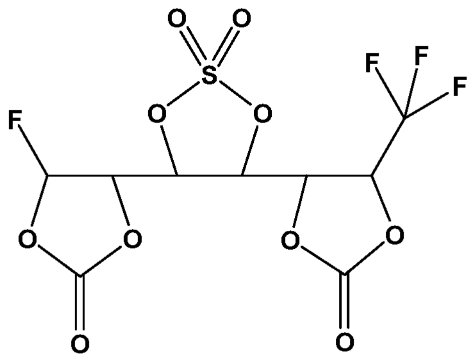

Compound 30

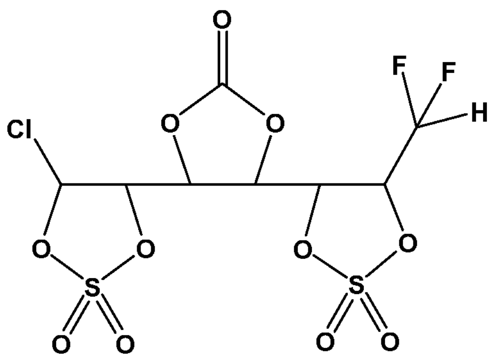

Compound 31

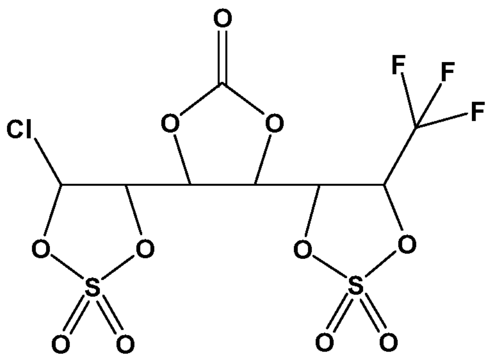

Compound 32

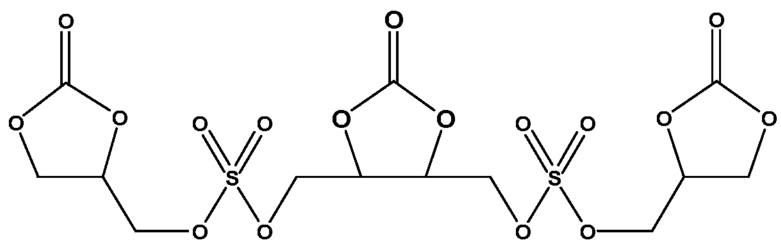

Compound 33

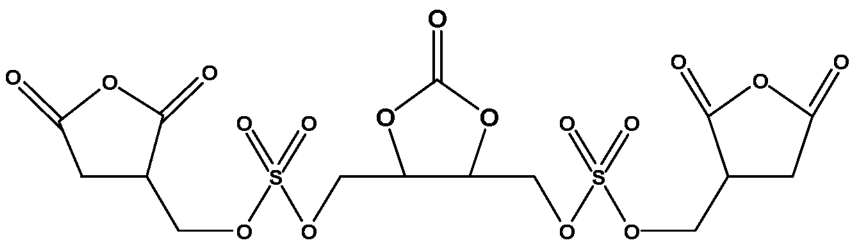

Compound 34

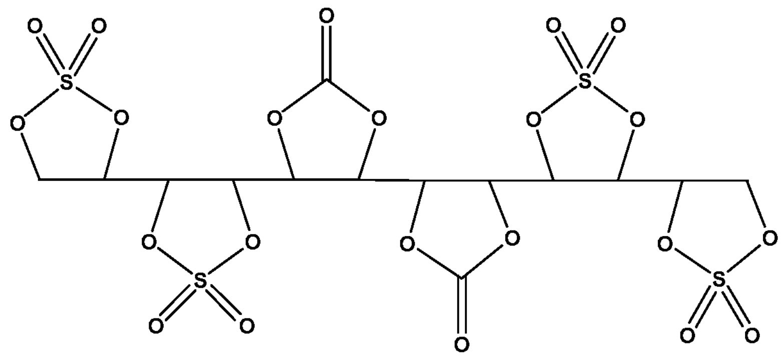

Compound 35

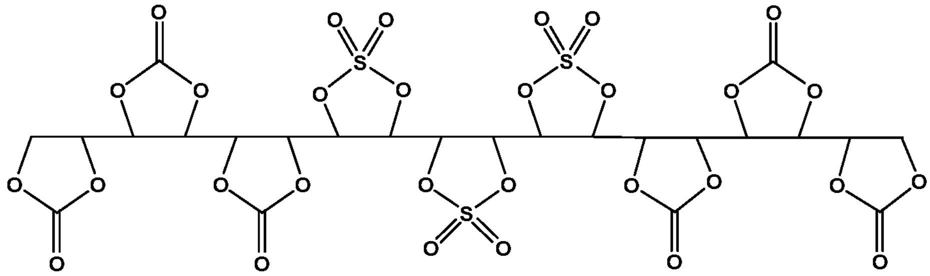

Compound 36

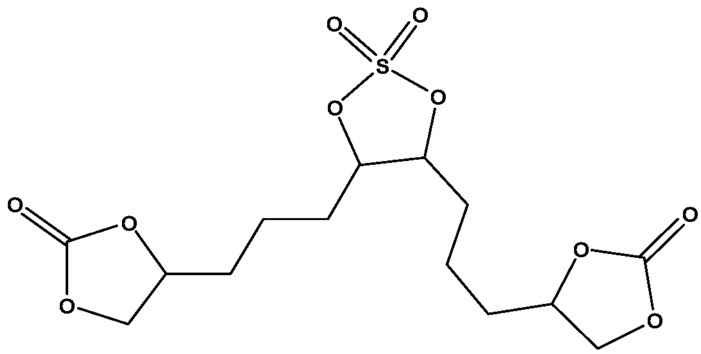

Compound 37

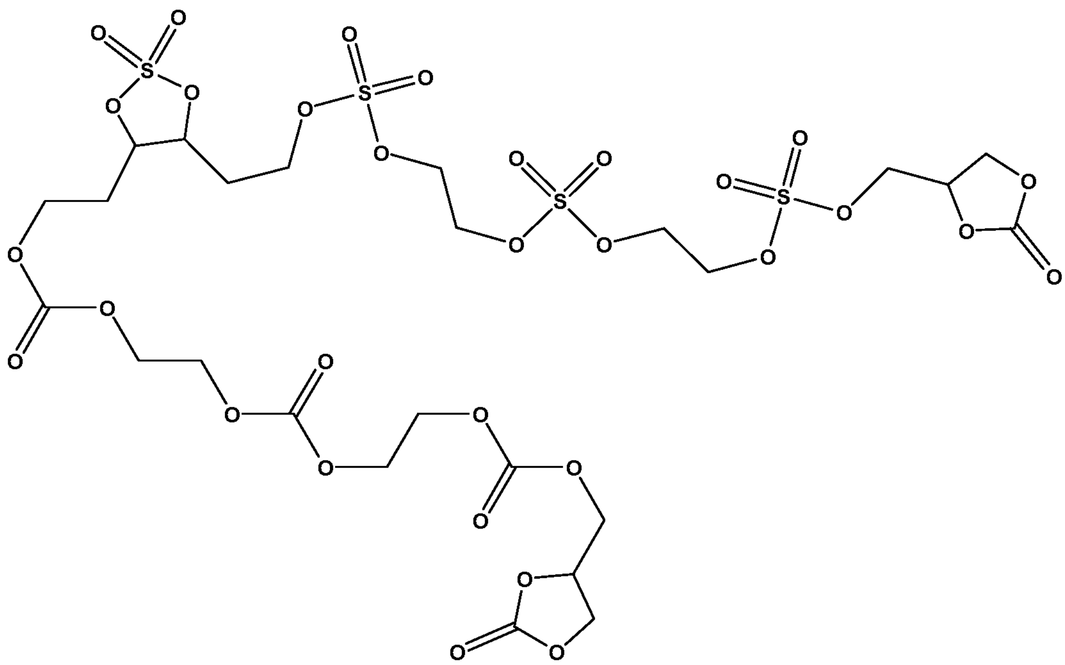

Compound 38

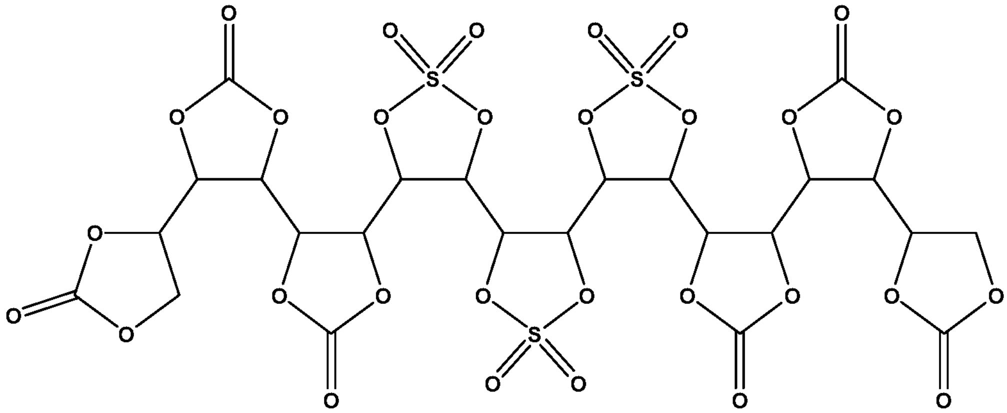

Compound 39

and

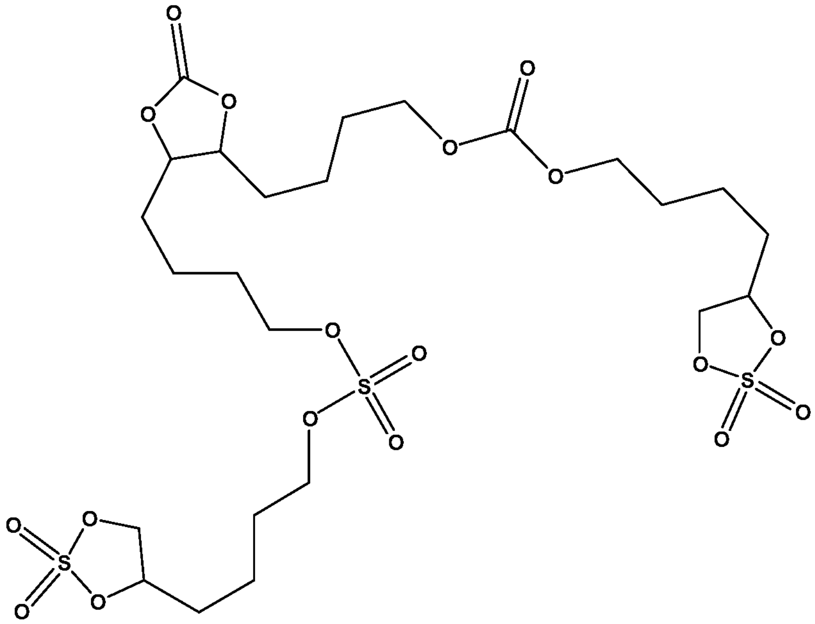

Compound 40.

Optionally, the positive electrode active material is selected from one or more of $LiNi_{0.5}CO_{0.2}Mn_{0.3}O_2$, $LiNi_{0.6}Co_{0.2}Mn_{0.2}O_2$, $LiNi_{0.7}Co_{0.1}Mn_{0.2}O_2$, $LiNi_{0.8}Co_{0.15}Al_{0.05}O_2$, $LiNi_{0.8}Co_{0.1}Mn_{0.1}O_2$, $LiNi_{0.6}Mn_{0.4}O_2$, and $LiNi_{0.8}Mn_{0.2}O_2$.

Optionally, the non-aqueous electrolyte further includes an auxiliary additive including at least one of an unsaturated cyclic carbonate compound, a fluorinated cyclic carbonate compound, an aromatic additive, a fluorine-containing anisole compound, a dicarboxylic acid anhydride, lithium difluorophosphate, and lithium bisfluorosulfonimide (LiFSI).

According to the lithium-ion battery provided in the present disclosure, the inventors found that when the compound represented by the formula 1 is used in combination with a high-nickel ternary material, a ternary high-nickel lithium-ion battery with an increased capacity retention rate during the high-temperature cycling and a lower gas generation during the cycling can be obtained. The compound represented by the formula 1 decomposes on the surface of the positive electrode to form a protective film, which uniformly covers the surface of the positive electrode material. On the one hand, it inhibits the decomposition of original alkaline oxides such as lithium carbonate on the surface of the positive electrode material to reduce the gas generation. On the other hand, it can well protect the structural stability of the positive electrode. It is speculated that the decomposition product of the formula 1 on the surface of the positive electrode forms a complex with nickel ions to form a relatively stable protective film, which limits its dissolution. At the same time, the formed film has certain elasticity. With the expansion and contraction of the positive electrode material,

the corresponding expansion and contraction will occur, so as to realize the protection of the positive electrode, and the film is not easy to break during the charge and discharge cycling.

## BRIEF DESCRIPTION OF THE DRAWINGS

FIG. 1 is a TEM image of a positive electrode after cycling provided in Example 7 of the present disclosure.
FIG. 2 is a TEM image of a positive electrode after cycling provided in Comparative Example 1 of the present disclosure.

## DETAILED DESCRIPTION

In order that the technical problems, technical solutions and advantageous effects to be solved by the present disclosure can be more clearly understood, the present disclosure will be described in further detail with reference to the accompanying drawings and the embodiments. It should be understood that the particular embodiments described herein are only used to explain the present disclosure, but are not intended to limit the present disclosure.

The embodiment of the present disclosure provides a lithium-ion battery, including a positive electrode, a negative electrode and a non-aqueous electrolyte. The positive electrode includes a positive electrode material layer. The positive electrode material layer includes a positive electrode active material. The positive electrode active material includes $LiNi_xCo_yMn_zL_{(1-x-y-z)}O_2$, where L is Al, Sr, Mg, Ti, Ca, Zr, Zn, Si, Cu, V or Fe, $0.5 \leq x \leq 1$, $0 \leq y \leq 0.5$, $0 \leq z \leq 0.5$, $0 \leq x + y + z \leq 1$. An upper limit voltage of the lithium-ion battery is ≥ 4.2 V The non-aqueous electrolyte includes a solvent, an electrolyte salt and a compound represented by formula 1:

A-D-B-E-C    Formula 1

where A, B, and C are each independently selected from a group containing a cyclic carbonate group, a cyclic sulfate group, a cyclic sulfite group, a cyclic sulfonate group, a cyclic sulfone group, a cyclic sulfoxide group, a cyclic carboxylate group or a cyclic anhydride group;
D and E are each independently selected from a single bond, or a group containing a hydrocarbylene group, an ether bond, a sulfur-oxygen double bond or a carbon-oxygen double bond.

Based on a total mass of the non-aqueous electrolyte as 100%, the compound represented by the formula 1 is added in an amount of 0.01 to 5.0%.

In the lithium-ion battery provided in the present disclosure, the compound represented by the formula 1 decomposes on the surface of the positive electrode to form a protective film, which uniformly covers the surface of the positive electrode material. On the one hand, it inhibits the decomposition of original alkaline oxides such as lithium carbonate on the surface of the positive electrode material to reduce a gas generation. On the other hand, it can well protect the structural stability of the positive electrode. It is speculated that the decomposition product of the formula 1 on the surface of the positive electrode forms a complex with nickel ions to form a relatively stable protective film, which limits its dissolution. At the same time, the formed film has certain elasticity. With the expansion and contraction of the positive electrode material, the corresponding expansion and contraction will occur, so as to realize the protection of the positive electrode, and the film is not easy to break during the charge and discharge cycling.

It should be noted that the performance of the battery is related to the content of nickel in the positive electrode active material and the content of the compound represented by the formula 1 in the non-aqueous electrolyte. When the nickel content in the positive electrode active material is too small, although adding the compound represented by the formula 1 can improve the high-temperature cycling performance of the battery to a certain extent, its improvement effect is relatively low. However, when the nickel content in the positive electrode active material is high ($0.5 \leq x \leq 1$), the compound represented by the formula 1 has an extremely excellent improvement effect on the high-temperature cycling performance of the battery, indicating that the presence of nickel in the positive electrode active material has a clear relationship with the compound represented by the formula 1. As the nickel content increases, the more the compound represented by the formula 1 can play its role in improving the battery. At the same time, when the addition amount of the compound represented by the formula 1 is too small, the film-forming protective effect cannot be achieved, and the improvement effect on the battery performance is not apparent. When the addition amount of the compound represented by the formula 1 is too large, not only will the film become too thick and the impedance increase, but also the viscosity of the electrolyte will significantly increase, which will affect the performance of the battery. Therefore, only by adding an appropriate amount of the compound represented by the formula 1, can it have a better matching effect

with the high-nickel ternary material.

When the charging upper limit voltage of the lithium-ion battery is higher, its electrolyte is more likely to decompose, and the compound represented by the formula 1 can effectively inhibit the decomposition of the electrolyte under high-voltage conditions, so it is especially suitable for a high-voltage lithium-ion battery with an upper limit voltage ≥ 4.2 V

In some embodiments, x is selected from 0.5, 0.55, 0.6, 0.65, 0.7, 0.75, 0.8, 0.83, 0.85, 0.88, 0.90, or 0.95.

In some embodiments, the positive electrode active material is selected from one or more of $LiNi_{0.5}CO_{0.2}Mn_{0.3}O_2$, $LiNi_{0.6}Co_{0.2}Mn_{0.2}O_2$, $LiNi_{0.7}Co_{0.1}Mn_{0.2}O_2$, $LiNi_{0.8}Co_{0.15}Al_{0.05}O_2$, $LiNi_{0.8}Co_{0.1}Mn_{0.1}O_2$, $LiNi_{0.6}Mn_{0.4}O_2$, and $LiNi_{0.8}Mn_{0.2}O_2$.

In some embodiments, the positive electrode active material incorporates element L by means of doping, and L is Al, Sr, Mg, Ti, Ca, Zr, Zn, Si, Cu, V or Fe. The doped element L can provide stronger A-O chemical bonds than Ni, Co, Mn and other active transition metals, inhibit the precipitation of lattice oxygen under a high voltage, and improve the structural stability of the material.

In some embodiments, A, B, and C each independently contain 1 to 5 of the cyclic carbonate group(s), the cyclic sulfate group(s), the cyclic sulfite group(s), the cyclic sulfonate group(s), the cyclic sulfone group(s), the cyclic sulfoxide group(s), the cyclic carboxylate group(s) or the cyclic anhydride group(s), and a total number of the cyclic carbonate group(s), the cyclic sulfate group(s), the cyclic sulfite group(s), the cyclic sulfonate group(s), the cyclic sulfone group(s), the cyclic sulfoxide group(s), the cyclic carboxylate group(s) or the cyclic anhydride group(s) of A, B, and C is less than or equal to 10.

In some embodiments, A and C are each independently selected from a group represented by formula 2:

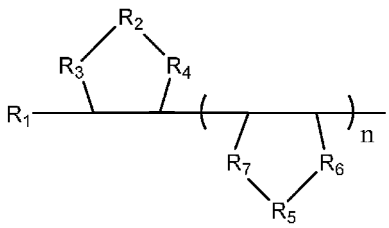

Formula 2

where n is selected from an integer of 0 to 4, and $R_1$ is selected from hydrogen, halogen, or a C1 to C5 halohydrocarbonyl group; $R_2$, $R_3$, $R_4$, $R_5$, $R_6$, and $R_7$ are each independently selected from a C1 to C3 hydrocarbylene group, a C1 to C3 alkoxy group, an oxygen atom,

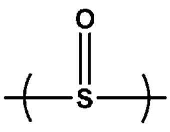,

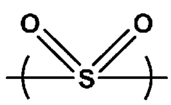, or 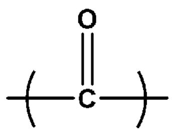,

in which at least one of $R_2$, $R_3$, and $R_4$ is selected from

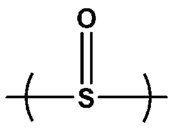,

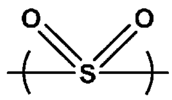, or 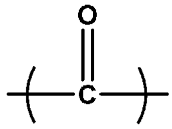,

and at least one of $R_2$, $R_3$, and $R_4$ is selected from the oxygen atom, and at least one of $R_5$, $R_6$, and $R_7$ is selected from

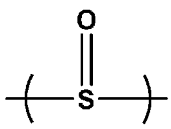, 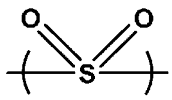, or 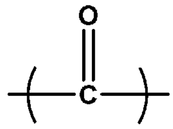,

and at least one of $R_5$, $R_6$, and $R_7$ is selected from the oxygen atom.

In preferred embodiments, a combined group of $-R_3-R_2-R_4-$ and a combined group of $-R_7-R_5-R_6-$ are each independently selected from

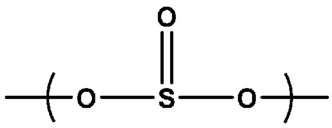, 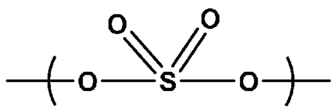,

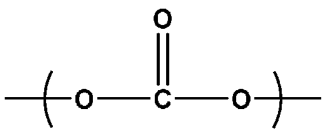, 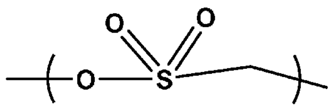, 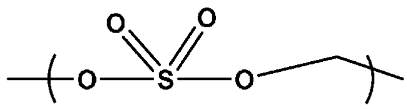,

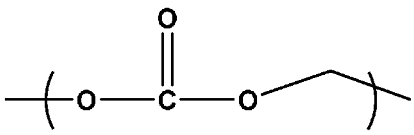 or 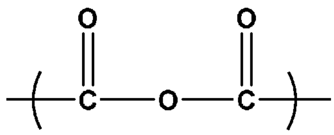.

In some embodiments, B is selected from a group represented by formula 3:

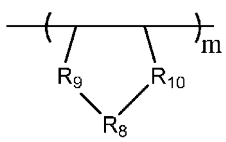

Formula 3

where m is selected from an integer of 1 to 4, and $R_8$, $R_9$, and $R_{10}$ are each independently selected from a C1 to C3 hydrocarbylene group, a C1 to C3 alkoxy group, an oxygen atom,

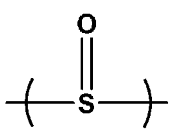, 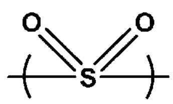, or 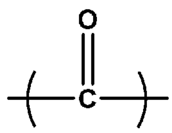,

at least one of $R_8$, $R_9$, and $R_{10}$ is selected from

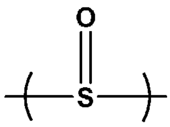, 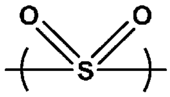, or 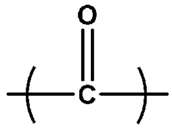,

and at least one of $R_8$, $R_9$, and $R_{10}$ is selected from the oxygen atom.

In preferred embodiments, a combined group of $-R_9-R_8-R_{10}-$ are each independently selected from

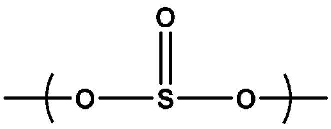, 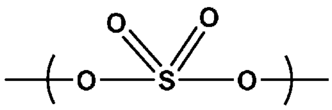, 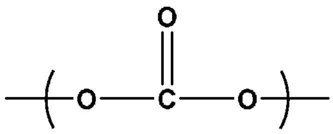,

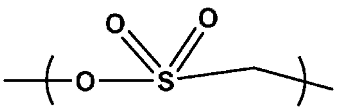, 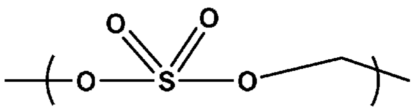, 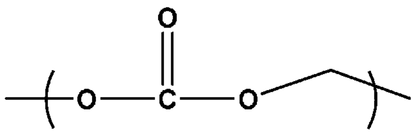

or

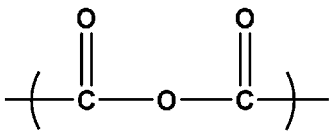.

In some embodiments, D and E are each independently selected from a group represented by formula 4:

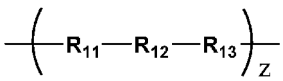

Formula 4

where z is selected from an integer of 0 to 4, $R_{11}$ and $R_{13}$ are each independently selected from a single bond or a C1 to C5 hydrocarbylene group, and $R_{12}$ is selected from a single bond,

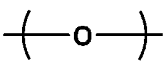, 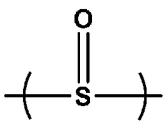, 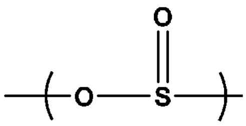, 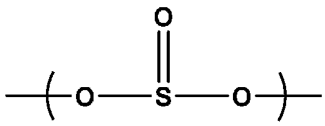, 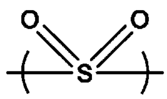,

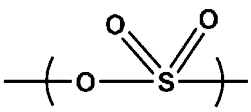, 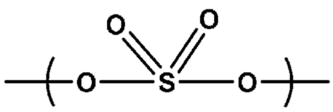, 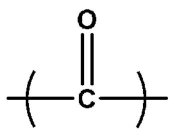, 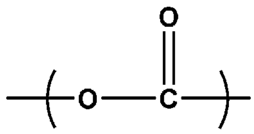

or

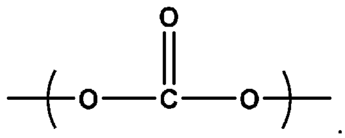

In some embodiments, A and C are same as each other, A and B are same as or different from each other, and D and E are same as each other.

When A and C are same as each other, and D and E are same as each other, the compound represented by the formula 1 has a symmetrical structure. Compared with an asymmetrical structure, the compound represented by the formula 1 of the symmetrical structure is more convenient in synthesis, and the yield of the product is higher, which is conducive to reducing the production cost.

In some embodiments, D and E are each independently selected from the single bond or the C1 to C5 hydrocarbylene group, and A, B, and C are each independently selected from the cyclic carbonate group, the cyclic sulfate group, the cyclic sulfite group, the cyclic sulfonate group, the cyclic sulfone group, the cyclic sulfoxide group, the cyclic carboxylate group or the cyclic anhydride group, which is substituted or unsubstituted. Preferably, when A, B or C is substituted, a substituent is selected from halogen, a hydrocarbonyl group or a halohydrocarbonyl group. More preferably, when A, B or C is substituted, the substituent is selected from halogen, an alkyl group or a haloalkyl group.

As an example, the compound represented by the formula 1 can be selected from one or more of following compounds:

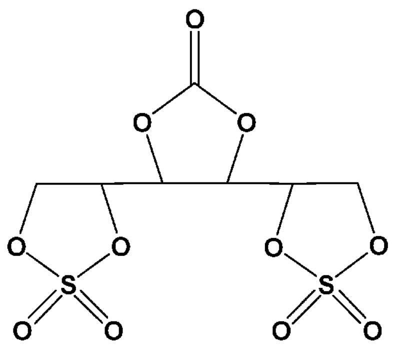

Compound 1

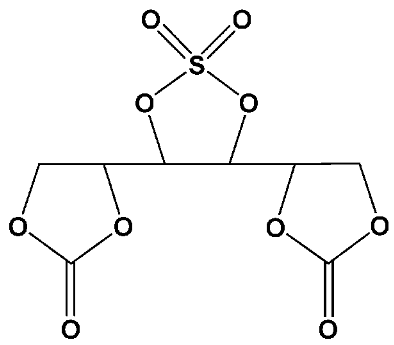

Compound 2

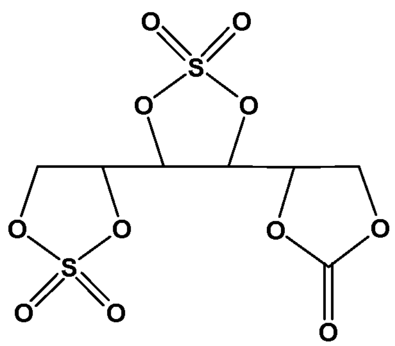

Compound 3

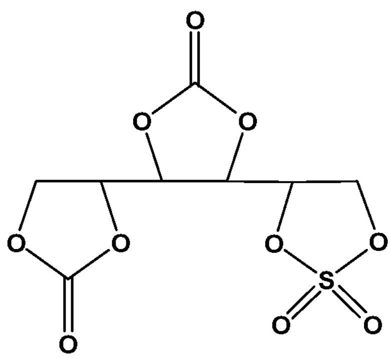

Compound 4

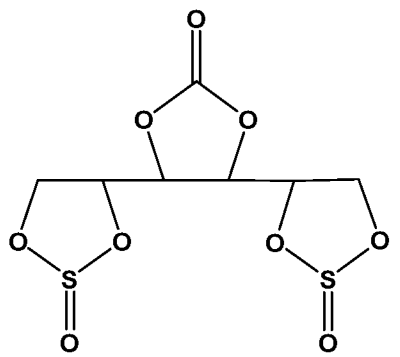

Compound 5

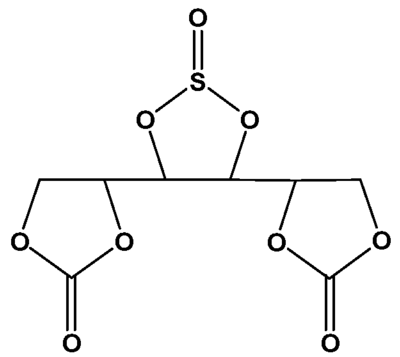

Compound 6

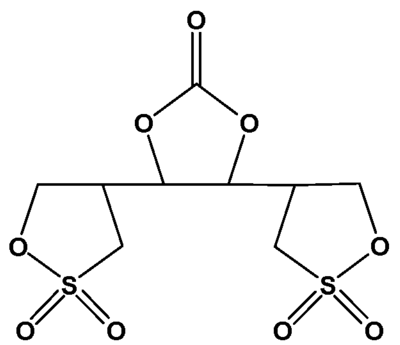

Compound 7

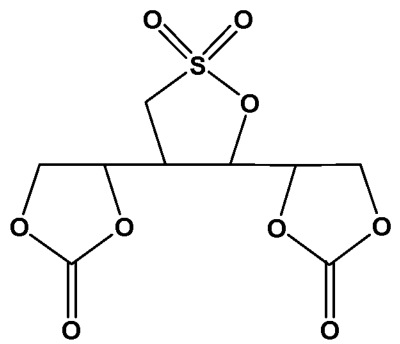

Compound 8

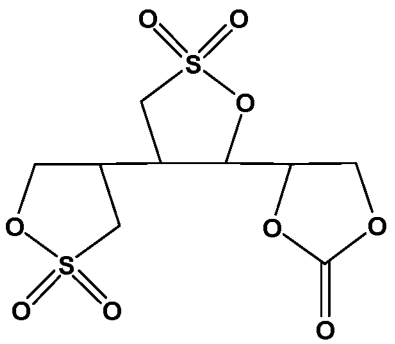

Compound 9

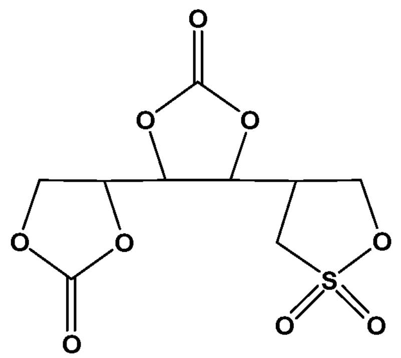

Compound 10

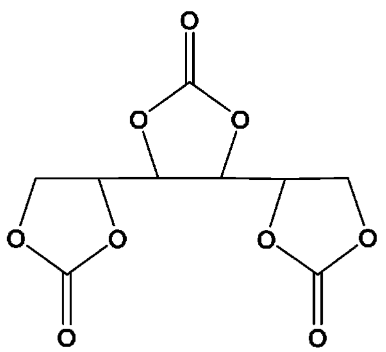

Compound 11

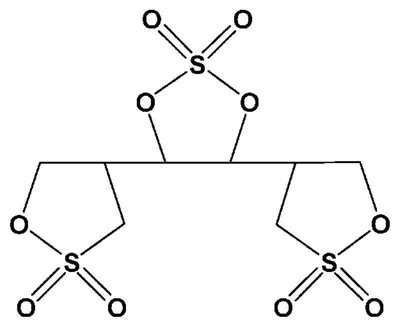

Compound 12

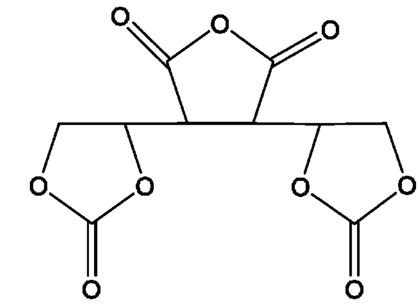

Compound 13

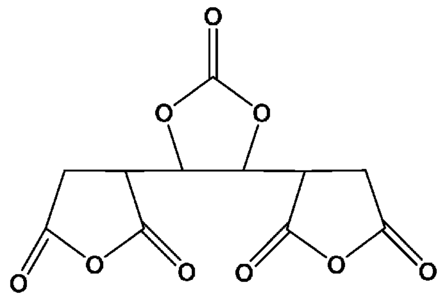

Compound 14

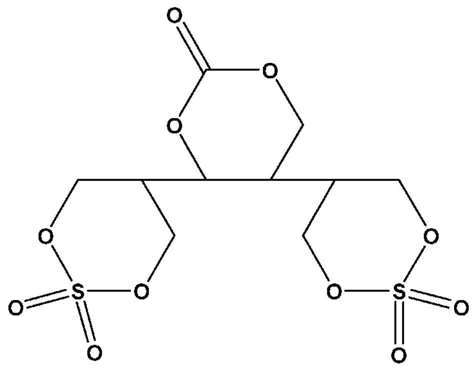

Compound 15

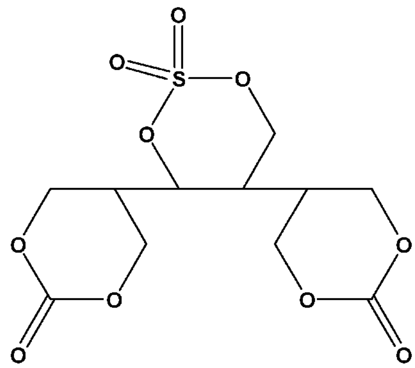

Compound 16

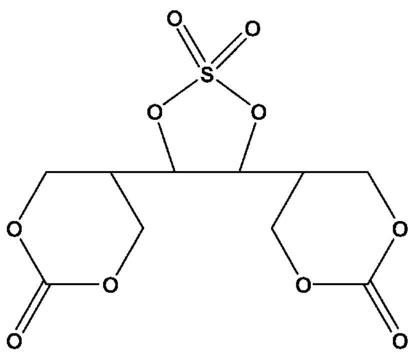

Compound 17

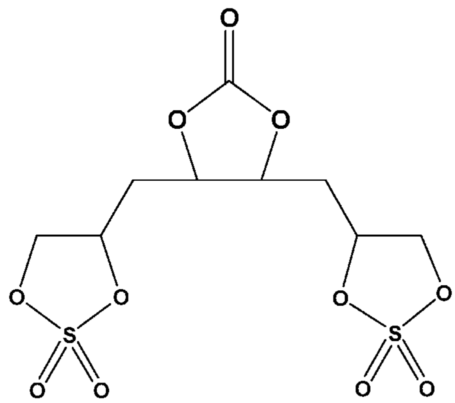

Compound 18

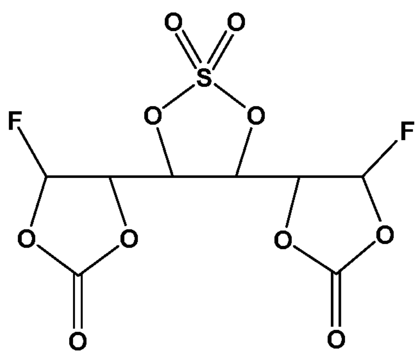

Compound 27

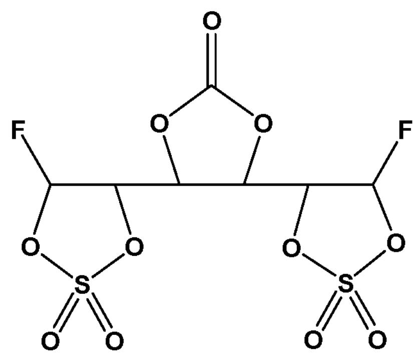

Compound 28

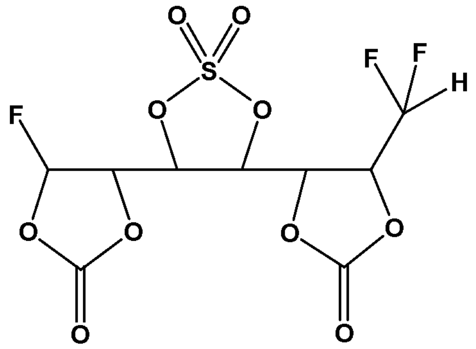

Compound 29

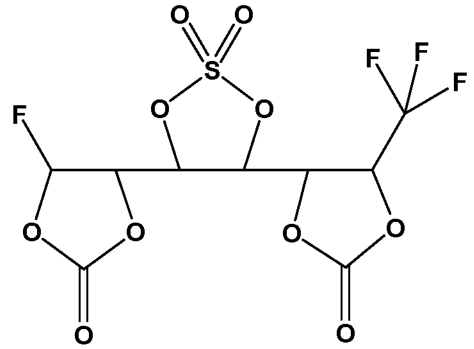

Compound 30

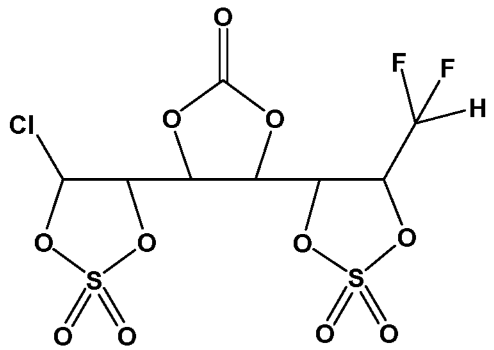

Compound 31

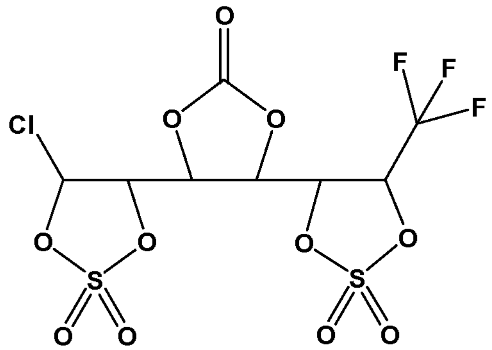

Compound 32

and

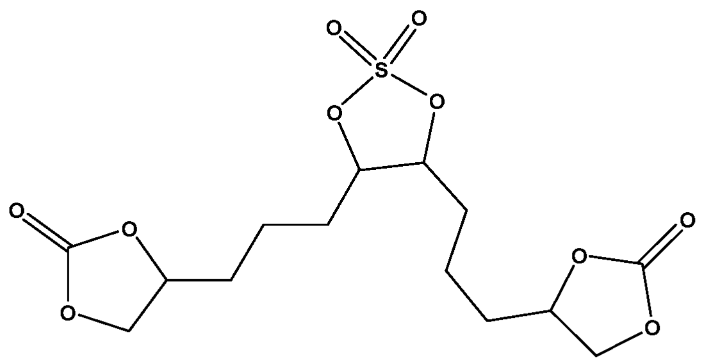

Compound 37.

In some embodiments, D and E are each independently selected from a group represented by formula 4:

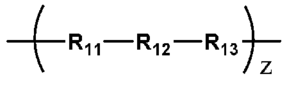

Formula 4

where z is selected from an integer of 1 to 4, $R_{11}$ and $R_{13}$ are each independently selected from a single bond or a C1 to C5 hydrocarbylene group, and $R_{12}$ is selected from

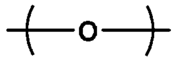,

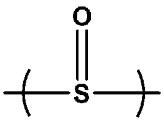, 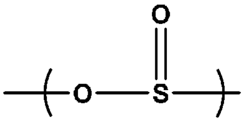, 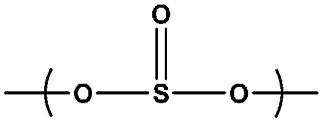, 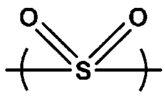, 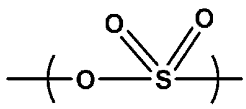,

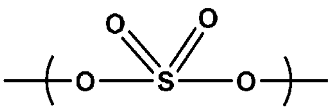, 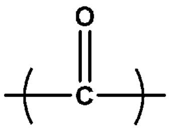, 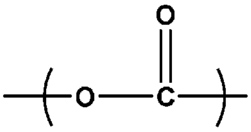 or 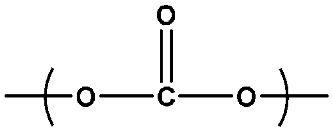;

A, B, and C are each independently selected from the cyclic carbonate group, the cyclic sulfate group, the cyclic sulfite group, the cyclic sulfonate group, the cyclic sulfone group, the cyclic sulfoxide group, the cyclic carboxylate group or the cyclic anhydride group, which is substituted or unsubstituted. Preferably, when A, B or C is substituted, a substituent is selected from halogen, a hydrocarbonyl group or a halohydrocarbonyl group. More preferably, when A, B or C is substituted, the substituent is selected from halogen, an alkyl group or a haloalkyl group.

As an example, the compound represented by the formula 1 can be selected from one or more of following compounds:

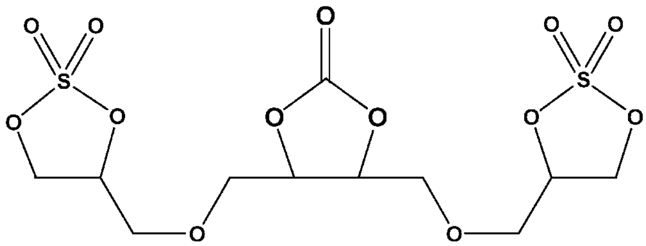

Compound 19

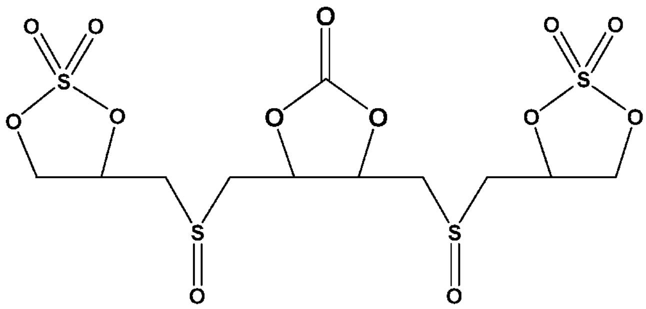

Compound 20

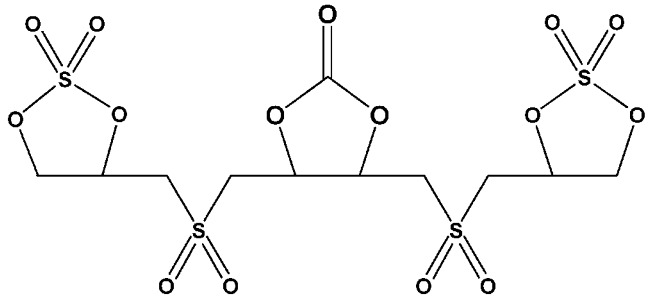

Compound 21

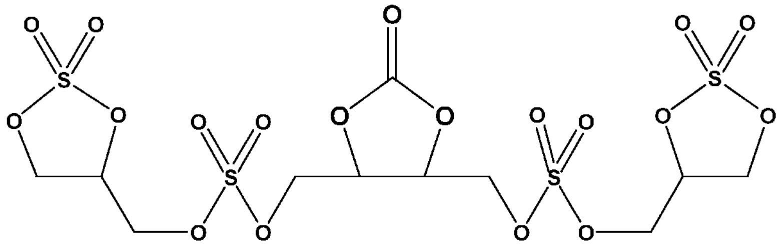

Compound 22

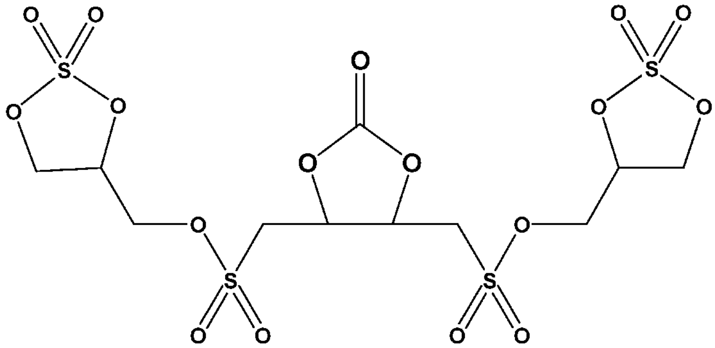

Compound 23

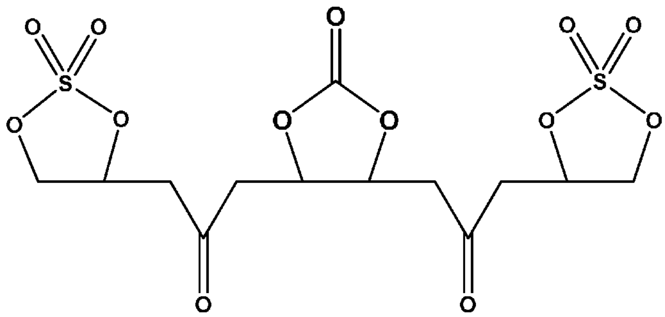

Compound 24

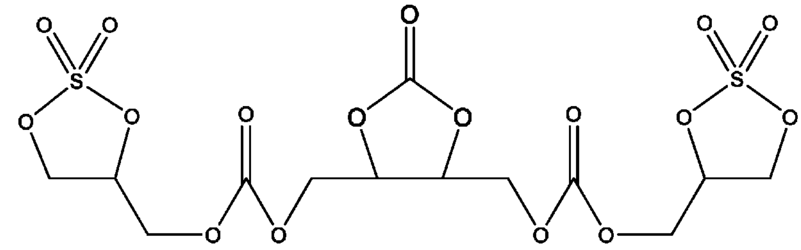

Compound 25

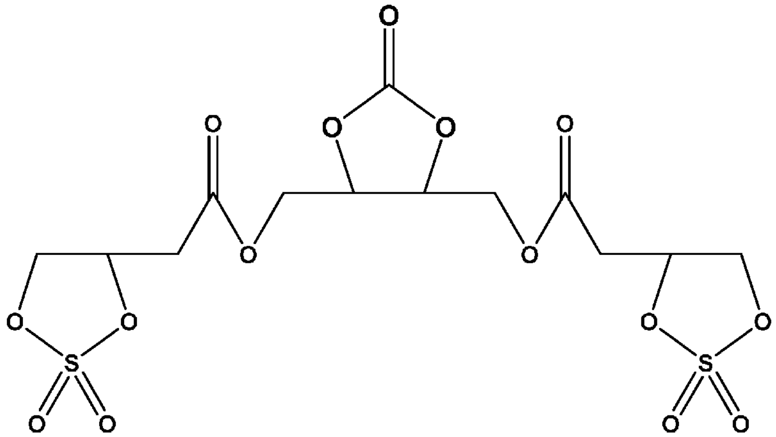

Compound 26

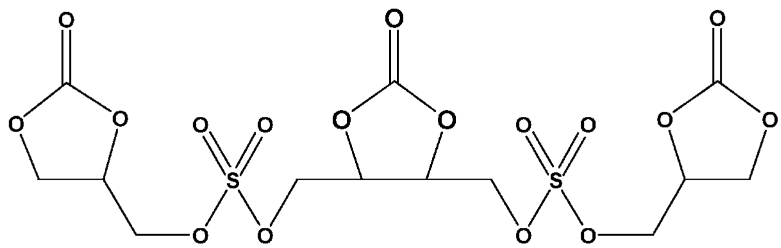

Compound 33

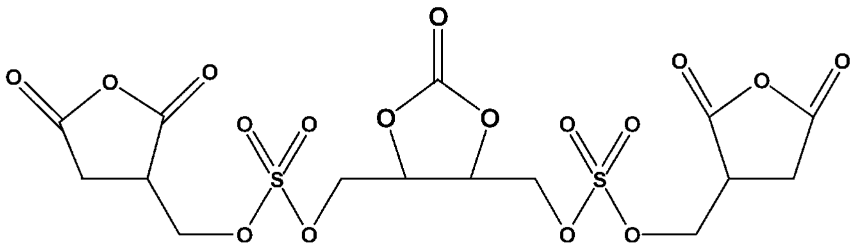

Compound 34

and

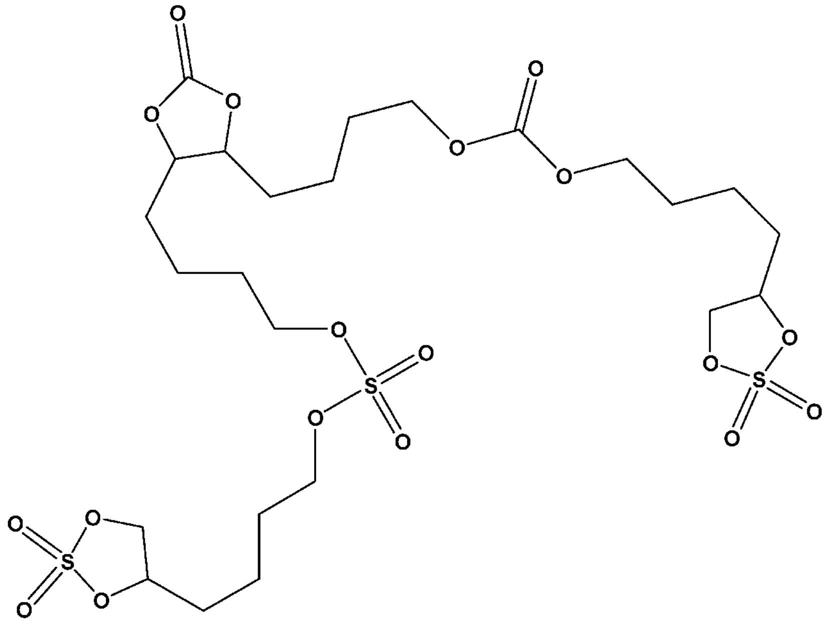

Compound 40.

In some embodiments, the compound represented by the formula 1 can also be selected from one or more of the following compounds:

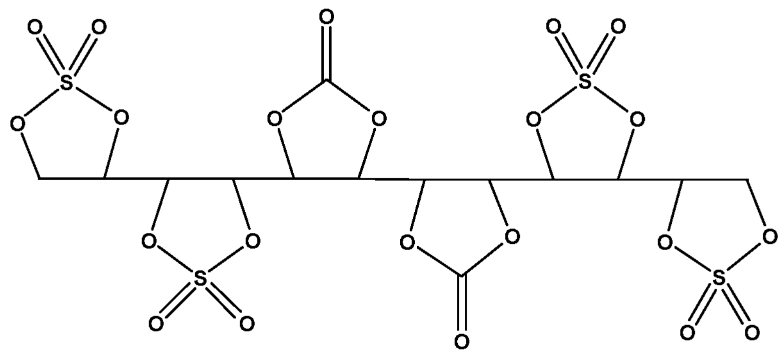

Compound 35

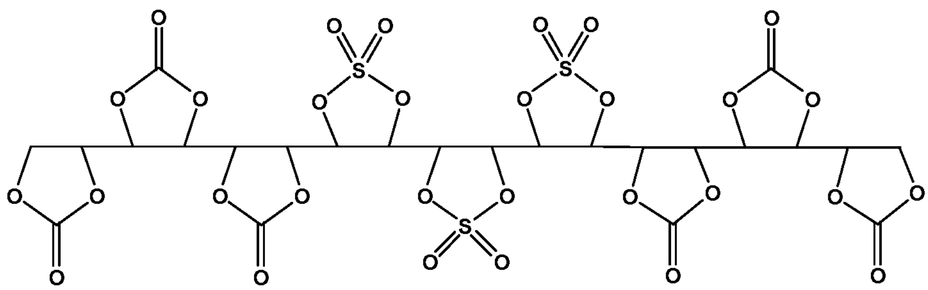

Compound 36

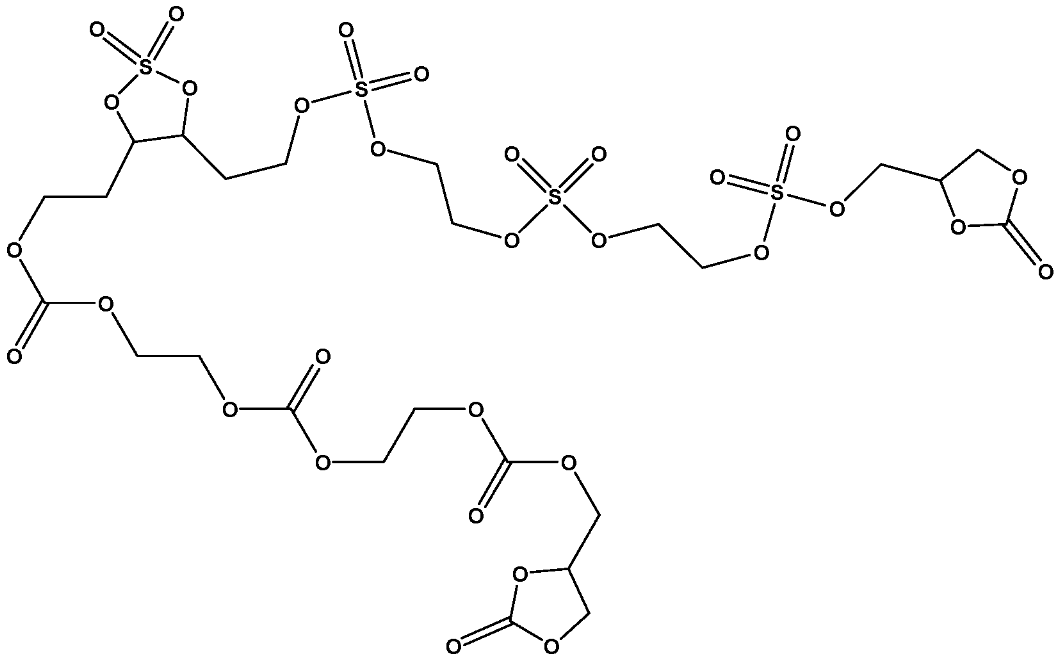

Compound 38

and

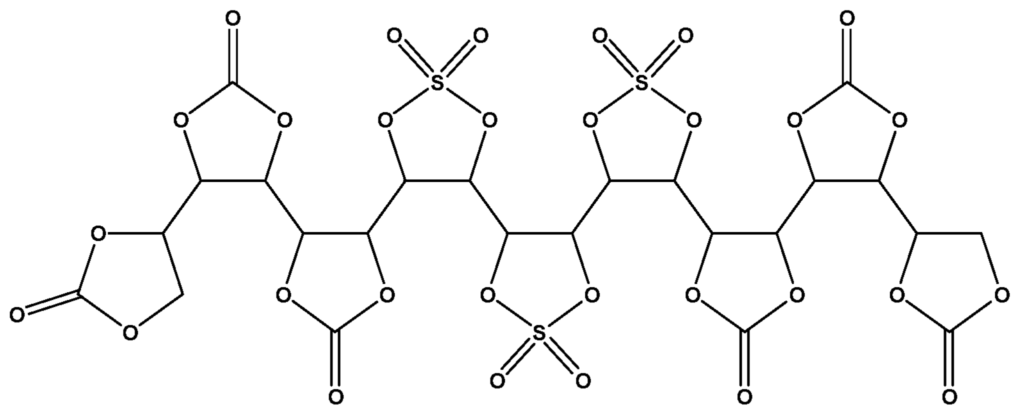

Compound 39.

It should be noted that the above are some of the compounds claimed in the present disclosure, but are not limited thereto, and should not be construed as limiting the present disclosure.

Those skilled in the art can know the preparation method of the above-mentioned compound according to the general knowledge in the field of chemical synthesis if they know the formula of the compound of the formula 1. For example:

Compound 1 can be prepared by a method as follows:

Organic solvents such as sorbitol, dimethyl carbonate, methanol alkaline substance catalyst potassium hydroxide, and DMF are placed in a reaction vessel, and reacted for several hours under heating conditions. A certain amount of oxalic acid is added to adjust the pH to neutrality. After filtering and recrystallization, an intermediate product 1 can be obtained. Then, the intermediate product 1, carbonate, thionyl chloride, etc. are esterified under high-temperature conditions to obtain an intermediate product 2, and then the intermediate product 2 is oxidized using an oxidizing agent such as sodium periodate to obtain the compound 1.

Compound 2 can be prepared by a method as follows:

Diacetone-D-mannitol, dimethyl carbonate, methanol, potassium carbonate, dioxane, etc. are reacted under heating and stirring for several hours. Then, a certain amount of oxalic acid is added to adjust the pH of the solution to neutrality. After filtering and concentrating, an intermediate product 3 is obtained. An appropriate amount of pure water,

carbonate, acid, etc. are added to the intermediate product 3 to perform a hydrolysis reaction to obtain an intermediate product 4. Then, the intermediate product 4, thionyl chloride and carbonate solvent are reacted under heating conditions to obtain an intermediate product 5. Finally, the intermediate product 5 is oxidized using an oxidizing agent such as sodium periodate to obtain the compound 2.

In some embodiments, the solvent includes one or more of ether solvents, nitrile solvents, carbonate solvents and carboxylate solvents.

Ether solvents include cyclic ethers or linear ethers.

Examples of cyclic ethers include one or more of 1,3-dioxolane (DOL), 1,4-dioxane (DX), crown ethers, tetrahydrofuran (THF), 2-methyltetrahydrofuran (2-$CH_3$-THF), and 2-trifluoromethyltetrahydrofuran (2-$CF_3$-THF).

Examples of linear ethers include one or more of dimethoxymethane (DMM), 1,2-dimethoxyethane (DME), and diglyme (TEGDME).

Examples of nitrile solvents include one or more of acetonitrile, glutaronitrile, and malononitrile.

Carbonate solvents include cyclic carbonates or linear carbonates.

Examples of cyclic carbonates include one or more of ethylene carbonate (EC), propylene carbonate (PC), γ-butyrolactone (GBL), and butylene carbonate (BC).

Examples of linear carbonates include one or more of dimethyl carbonate (DMC), ethyl methyl carbonate (EMC), diethyl carbonate (DEC), methyl n-propyl carbonate, ethyl n-propyl carbonate, and dipropyl carbonate (DPC).

Carboxylate solvents include cyclic carboxylates or linear carbonates.

Examples of cyclic carboxylates include one or more of γ-butyrolactone, γ-valerolactone, and δ-valerolactone.

Examples of linear carbonates include one or more of methyl acetate (MA), ethyl acetate (EA), propyl acetate (EP), butyl acetate, propyl propionate (PP), and butyl propionate.

In some embodiments, the electrolyte salt is selected from a lithium salt. In preferred embodiments, the electrolyte salt is selected from one or more of $LiPF_6$, $LiBF_4$, LiBOB, LiDFOB, $LiN(SO_2CF_3)_2$, $LiN(SO_2C_2F_5)_2$, $LiC(SO_2CF_3)_3$, $LiN(SO_2F)_2$, $LiClO_4$, $LiAsF_6$, $LiSbF_6$, $LiCF_3SO_3$, $Li_2B_{10}Cl_{10}$, a lithium salt of a lower aliphatic acid, and $LiAlCl_4$.

In some embodiments, the concentration of the electrolyte salt in the non-aqueous electrolyte is from 0.1 mol/L to 8 mol/L.

In preferred embodiments, the concentration of the electrolyte salt in the non-aqueous electrolyte is from 0.5 mol/L to 4 mol/L. Specifically, the concentration of the electrolyte salt may be 0.5 mol/L, 1 mol/L, 1.5 mol/L, 2 mol/L, 2.5 mol/L, 3 mol/L, 3.5 mol/L or 4 mol/L.

In some embodiments, the non-aqueous electrolyte further includes an auxiliary additive including at least one of an unsaturated cyclic carbonate compound, a fluorinated cyclic carbonate compound, an aromatic additive, a fluorine-containing anisole compound, a dicarboxylic acid anhydride, lithium difluorophosphate, and lithium bis(fluorosulfonyl)imide (LiFSI).

In some embodiments, the unsaturated cyclic carbonate compound includes at least one of vinylene carbonate (VC), vinyl ethylene carbonate (VEC), 4,5-dimethyl vinylene carbonate, phenyl vinylene carbonate and 4,5-divinyl ethylene carbonate.

When the non-aqueous electrolyte contains the unsaturated cyclic carbonate compound, based on the total mass of the non-aqueous electrolyte as 100%, the content of the unsaturated cyclic carbonate compound is from 0.1 to 5%.

In some embodiments, the fluorinated cyclic carbonate compound includes one or more of fluoroethylene carbonate (FEC), 4,4-difluoroethylene carbonate, 4,5-difluoroethylene carbonate, 4-fluoro-4-methyl ethylene carbonate, 4,5-difluoro-4-methyl ethylene carbonate, 4-fluoro-5-methyl ethylene carbonate, 4,4-difluoro-5-methyl ethylene carbonate, 4-(fluoromethyl) ethylene carbonate, 4-(difluoromethyl)ethylene carbonate, 4-(trifluoromethyl)ethylene carbonate, 4-(fluoromethyl)-4-fluoroethylene carbonate, 4-(fluoromethyl)-5-fluoroethylene carbonate, 4-fluoro-4,5-dimethyl ethylene carbonate, 4,5-difluoro-4,5-dimethyl ethylene carbonate and 4,4-difluoro-5,5-dimethyl ethylene carbonate.

When the non-aqueous electrolyte contains the fluorinated cyclic carbonate compound, based on the total mass of the non-aqueous electrolyte as 100%, the content of the fluorinated cyclic carbonate compound is from 0.1 to 30%.

In some embodiments, the aromatic additive includes one or more of biphenyl, alkylbiphenyl, terphenyl, partially hydrogenated terphenyl, cyclohexylbenzene, tert-butylbenzene, tert-amylbenzene, diphenyl ether, dibenzofuran and other aromatic compounds; 2-fluorobiphenyl, o-cyclohexylfluorobenzene, and p-cyclohexylfluorobenzene.

When the non-aqueous electrolyte contains the aromatic additive, based on the total mass of the non-aqueous electrolyte as 100%, the mass percent content of the aromatic additive is from 0.1 to 5%.

In some embodiments, the fluorine-containing anisole compound includes one or more of 2,4-difluoroanisole, 2,5-difluoroanisole, and 2,6-difluoroanisole. When the non-aqueous electrolyte contains the fluorine-containing anisole compound, based on the total mass of the non-aqueous electrolyte as 100%, the mass percent content of the fluorine-containing anisole compound is from 0.1 to 5%.

In some embodiments, the dicarboxylic acid anhydride includes one or more of succinic acid, maleic acid, and phthalic acid. When the non-aqueous electrolytic solution contains the dicarboxylic acid anhydride, based on the total mass of the non-aqueous electrolyte as 100%, the mass percent content of the dicarboxylic acid anhydride is from 0.1

to 5%.

In some embodiments, when the non-aqueous electrolyte contains lithium difluorophosphate, based on the total mass of the non-aqueous electrolyte as 100%, the mass percent content of lithium difluorophosphate is from 0.1 to 2%.

In some embodiments, when the non-aqueous electrolyte contains lithium bis(fluorosulfonyl)imide (LiFSI), based on the total mass of the non-aqueous electrolyte as 100%, the mass percent content of lithium bis(fluorosulfonyl)imide (LiFSI) is from 0.1 to 5%.

In some embodiments, the auxiliary additive also includes nitrogen-containing compounds such as 1-methyl-2-pyrrolidone, 1-methyl-2-piperidone, 3-methyl-2-oxazolidinone, 1,3-dimethyl-2-imidazolidinone, and N-methyl succinimide; hydrocarbon compounds such as heptane, octane and cycloheptane; and fluorine-containing aromatic compounds such as fluorobenzene, difluorobenzene and trifluorotoluene.

It should be noted that, unless otherwise specified, in general, the mass percent content range of any optional substance in the non-aqueous electrolyte in the auxiliary additive is 10% or less, preferably, the mass percent content is from 0.1 to 5%.

In preferred embodiments, the positive electrode active material is selected from one or more of $LiNi_{0.5}Co_{0.2}Mn_{0.3}O_2$, $LiNi_{0.6}Co_{0.2}Mn_{0.2}O_2$, $LiNi_{0.7}Co_{0.1}Mn_{0.2}O_2$, $LiNi_{0.8}Co_{0.1}Mn_{0.1}O_2$, $LiNi_{0.6}Mn_{0.4}O_2$, and $LiNi_{0.8}Mn_{0.2}O_2$.

In some embodiments, the positive electrode further includes a positive electrode current collector, and the positive electrode material layer covers a surface of the positive electrode current collector. The positive electrode current collector is selected from metal materials that can conduct electrons. Preferably, the positive electrode current collector includes one or more of Al, Ni, and stainless steel. In more preferred embodiments, the positive current collector is selected from aluminum foil.

In some embodiments, the positive electrode material layer further includes a positive electrode binder and a positive electrode conductive agent. The positive electrode active material, the positive electrode binder and the positive electrode conductive agent are blended to obtain the positive electrode material layer.

The positive electrode binder includes one or more of thermoplastic resins such as polyvinylidene fluoride, vinylidene fluoride copolymers, polytetrafluoroethylene, vinylidene fluoride-hexafluoropropylene copolymers, tetrafluoroethylene-hexafluoropropylene copolymers, tetrafluoroethylene-perfluoroalkyl vinyl ether copolymers, ethylene-tetrafluoroethylene copolymers, vinylidene fluoride-tetrafluoroethylene copolymers, vinylidene fluoride-trifluoroethylene copolymers, vinylidene fluoride-trichloroethylene copolymers, vinylidene fluoride-fluoroethylene copolymers, vinylidene fluoride-hexafluoropropylene-tetrafluoroethylene copolymers, thermoplastic polyimides, polyethylene, polypropylene and so on; acrylic resins; and styrene butadiene rubbers.

The positive electrode conductive agent includes one or more of conductive carbon black, conductive carbon spheres, conductive graphite, conductive carbon fibers, carbon nanotubes, graphene or reduced graphene oxide.

In some embodiments, the negative electrode includes a negative electrode material layer including a negative electrode active material. The negative electrode active material includes one or more of a silicon-based negative electrode, a carbon-based negative electrode, a tin-based negative electrode, and a lithium negative electrode. The silicon-based negative electrode includes one or more of silicon materials, silicon oxides, silicon-carbon composite materials, and silicon alloy materials. The carbon-based negative electrode includes one or more of graphite, hard carbon, soft carbon, graphene, and intermediate phase carbon microspheres. The tin-based negative electrode includes one or more of tin, tin carbon, tin oxide, and tin metal compounds. The lithium negative electrode includes one or more of metal lithium or a lithium alloy. The lithium alloy may specifically be at least one of a lithium-silicon alloy, a lithium-sodium alloy, a lithium-potassium alloy, a lithium-aluminum alloy, a lithium-tin alloy and a lithium-indium alloy.

In some embodiments, the negative electrode further includes a negative electrode current collector, and the negative electrode material layer covers a surface of the negative electrode current collector. The negative electrode current collector is selected from metal materials that can conduct electrons. Preferably, the negative electrode current collector includes one or more of Cu, Ni, and stainless steel. In more preferred embodiments, the negative current collector is selected from aluminum foil.

In some embodiments, the negative electrode material layer further includes a negative electrode binder and a negative electrode conductive agent. The negative electrode active material, the negative electrode binder and the negative electrode conductive agent are blended to obtain the negative electrode material layer.

The negative electrode binder includes one or more of thermoplastic resins such as polyvinylidene fluoride, vinylidene fluoride copolymers, polytetrafluoroethylene, vinylidene fluoride-hexafluoropropylene copolymers, tetrafluoroethylene-hexafluoropropylene copolymers, tetrafluoroethylene-perfluoroalkyl vinyl ether copolymers, ethylene-tetrafluoroethylene copolymers, vinylidene fluoride-tetrafluoroethylene copolymers, vinylidene fluoride-trifluoroethylene copolymers, vinylidene fluoride-trichloroethylene copolymers, vinylidene fluoride-fluoroethylene copolymers, vinylidene fluoride-hexafluoropropylene-tetrafluoroethylene copolymers, thermoplastic polyimides, polyethylene, polypropylene and so on; acrylic resins; and styrene butadiene rubbers.

The negative electrode conductive agent includes one or more of conductive carbon black, conductive carbon spheres, conductive graphite, conductive carbon fibers, carbon nanotubes, graphene or reduced graphene oxide.
In some embodiments, the battery further includes a separator, and the separator is located between the positive electrode and the negative electrode.
The separator can be an existing conventional separator, which can be a ceramic separator, a polymer separator, a non-woven fabric, an inorganic-organic composite separator, etc., including but not limited to separators such as single-layer PP (polypropylene), single-layer PE (polyethylene), double-layer PP/PE, double-layer PP/PP, and triple-layer PP/PE/PP separators, and the like.
The present disclosure is further illustrated by way of examples below.

1. Examples 1 to 42 and Comparative Examples 1 to 9

1) Preparation of electrolyte

Ethylene carbonate (EC), diethyl carbonate (DEC) and ethyl methyl carbonate (EMC) were mixed according to a mass ratio of EC:DEC:EMC=1:1:1. Then, lithium hexafluorophosphate ($LiPF_6$) was added to a molar concentration of 1 mol/L, and then each additive was added according to the following tables. The amount of the additive was calculated as a percent of the total mass of the electrolyte.

2) Preparation of positive electrode sheet

A positive active material, conductive carbon black Super-P and binder polyvinylidene fluoride (PVDF) were mixed at a mass ratio of 93:4:3, and then they were dispersed in N-methyl-2-pyrrolidone (NMP) to obtain a positive electrode slurry. The positive electrode active material is shown in the following tables. The positive electrode slurry was evenly coated on both sides of aluminum foil. After drying, calendering and vacuum drying, an aluminum lead-out wire was welded with an ultrasonic welder to obtain a positive electrode sheet with a thickness of 120 to 150 μm.

3) Preparation of negative electrode sheet

Negative electrode active material graphite, conductive carbon black Super-P, binder styrene-butadiene rubber (SBR) and carboxymethyl cellulose (CMC) were mixed at a mass ratio of 94:1:2.5:2.5, and then they were dispersed in deionized water to obtain a negative electrode slurry. The negative electrode slurry was coated on both sides of copper foil. After drying, calendering and vacuum drying, a nickel lead-out wire was welded with an ultrasonic welder to obtain a negative electrode sheet with a thickness of 120 to 150 μm.

4) Preparation of battery cell

A three-layer separator with a thickness of 20 μm was placed between the positive electrode sheet and the negative electrode sheet, and then a sandwich structure composed of the positive electrode sheet, the negative electrode sheet and the separator was wound. Then, the wound body was flattened and put into an aluminum foil packaging bag, and baked under vacuum at 75°C for 48 hours to obtain a battery cell to be filled with a liquid.

5) Injection and formation of battery cell

In a glove box with a water content of 20 ppm or less and an oxygen content of 50 ppm or less, the above-prepared electrolyte was injected into the battery cell, packaged under vacuum, and left standing at 45°C for 24 h.
Then, the conventional formation of the first charging was carried out according to the following steps: charging at a constant current of 0.05 C for 180 min, charging at a constant current of 0.1 C for 180 min, charging at a constant current of 0.2 C for 120 min, aging at 45°C for 48h, secondary sealing under vacuum, then further charging at a constant current of 0.2C to 4.4 V ($LiNi_{0.5}Co_{0.2}Mn_{0.3}O_2$/AG) or 4.2 V ($LiNi_{0.8}Co_{0.15}Al_{0.05}O_2$/AG) or 4.2 V ($LiNi_{0.8}Co_{0.1}Mn_{0.1}O_2$/AG) or 4.25 V ($LiNi_{0.7}Co_{0.1}Mn_{0.2}O_2$/AG) or 4.35 V ($LiNi_{0.6}Co_{0.2}Mn_{0.2}O_2$/AG), and discharging at a constant current of 0.2 C to 3.0 V

2. Performance testing

The lithium-ion batteries prepared in Examples 1 to 42 and Comparative Examples 1 to 9 were subjected to the following performance tests.
Testing of high-temperature cycling performance

The prepared lithium-ion battery was placed in an oven with a constant temperature of 45°C, charged at a constant current of 1C to 4.4 V ($LiNi_{0.5}Co_{0.2}Mn_{0.3}O_2$/AG) or 4.2 V ($LiNi_{0.8}Co_{0.15}Al_{0.05}O_2$/AG) or 4.2 V ($LiNi_{0.8}Co_{0.1}Mn_{0.1}O_2$/AG) or 4.25 V ($LiNi_{0.7}Co_{0.1}Mn_{0.2}O_2$/AG) or 4.35 V ($LiNi_{0.6}Co_{0.2}Mn_{0.2}O_2$/AG), then charged at a constant current and a constant voltage until the current drops to 0.05C, and then discharged at a constant current of 1C to 3.0 V, so as to circulate in this way. The first discharge capacity and the last discharge capacity were recorded, and the initial battery volume and the volume of the battery after 1000 cycles were measured.

The capacity retention rate during the high-temperature cycling was calculated according to the following formula:

$$\text{Capacity retention ratio} = \text{last discharge capacity} / \text{first discharge capacity} \times 100\%.$$

The volume expansion rate during the high-temperature cycling is calculated according to the following formula:

$$\text{Volume expansion rate (\%)} = (\text{battery volume after cycle} - \text{initial battery volume})/\text{initial battery volume} \times 100\%.$$

The batteries used in the following data tests are the same except for the differences listed in the tables.

1. The test results obtained in Examples 1 to 10 and Comparative Examples 1 to 4 are shown in Table 1.

Table 1

| Group | Positive electrode material | Compound represented by formula 1 and its mass content | 1000 cycles at 45°C at 1 C | |
|---|---|---|---|---|
| | | | Capacity retention rate | Volume expansion rate |
| Example 1 | $LiNi_{0.5}Co_{0.2}Mn_{0.3}O_2$ | Compound 1: 0.01% | 68.8% | 58.8% |
| Example 2 | $LiNi_{0.5}Co_{0.2}Mn_{0.3}O_2$ | Compound 1: 0.02% | 69.4% | 55.4% |
| Example 3 | $LiNi_{0.5}Co_{0.2}Mn_{0.3}O_2$ | Compound 1: 0.05% | 70.2% | 51.4% |
| Example 4 | $LiNi_{0.5}Co_{0.2}Mn_{0.3}O_2$ | Compound 1: 0.1% | 72.8% | 38.9% |
| Example 5 | $LiNi_{0.5}Co_{0.2}Mn_{0.3}O_2$ | Compound 1: 0.2% | 73.0% | 26.4% |
| Example 6 | $LiNi_{0.5}Co_{0.2}Mn_{0.3}O_2$ | Compound 1: 0.5% | 73.4% | 16.4% |
| Example 7 | $LiNi_{0.5}Co_{0.2}Mn_{0.3}O_2$ | Compound 1: 1% | 77.2% | 7.2% |
| Example 8 | $LiNi_{0.5}Co_{0.2}Mn_{0.3}O_2$ | Compound 1: 2% | 75.2% | 8.2% |
| Example 9 | $LiNi_{0.5}Co_{0.2}Mn_{0.3}O_2$ | Compound 1: 3% | 73.2% | 13.4% |
| Example 10 | $LiNi_{0.5}Co_{0.2}Mn_{0.3}O_2$ | Compound 1: 5% | 76.4% | 30.4% |
| Comparative Example 1 | $LiNi_{0.5}Co_{0.2}Mn_{0.3}O_2$ | - | 63.4% | 57.4% |
| Comparative Example 2 | $LiNi_{0.5}Co_{0.2}Mn_{0.3}O_2$ | Compound 1: 0.005% | 68.0% | 53.6% |
| Comparative Example 3 | $LiNi_{0.5}Co_{0.2}Mn_{0.3}O_2$ | Compound 1: 6% | 65.6% | 45.6% |

As can be seen from the test results in Table 1, when the positive electrode active material is $LiNi_{0.5}Co_{0.2}Mn_{0.3}O_2$, by comparing Examples 1 to 10 and Comparative Example 1, adding the compound represented by the formula 1 can significantly inhibit gas production during the high-temperature cycling. When the content of the compound represented by the formula 1 is from 1% to 2%, the volume expansion rate of the battery during the high-temperature cycling is at least 7.2% to 8.2%, and at this time has the best capacity retention rate during the high-temperature cycling of 75.2%

to 77.2%. Comparison of Examples 1 to 10 and Comparative Examples 2 and 3 show that when the content of the compound represented by the formula 1 is below 0.01% or above 5%, the gas production of the battery during the cycling increases significantly, and the high-temperature cycling performance also decreases significantly. This indicates that in the high-nickel ternary lithium-ion battery, the reasonable addition amount of the compound represented by the formula 1 is from 0.01% to 5%, too large or too small amount is not conducive to the improvement of the battery performance.

2. The test results obtained in Examples 7, 11 to 14 and Comparative Examples 1, 4 to 9 are shown in Table 2.

Table 2

| Group | Positive electrode material | Compound represented by formula 1 and its mass content | 1000 cycles at 45°C at 1C | |
|---|---|---|---|---|
| | | | Capacity retention rate | Volume expansion rate |
| Example 7 | $LiNi_{0.5}Co_{0.2}Mn_{0.3}O_2$ | Compound 1: 1% | 77.2% | 7.2% |
| Example 11 | $LiNi_{0.6}Co_{0.2}Mn_{0.2}O_2$ | Compound 1: 1% | 77.4% | 11.3% |
| Example 12 | $LiNi_{0.7}Co_{0.1}Mn_{0.2}O_2$ | Compound 1: 1% | 77.7% | 16.5% |
| Example 13 | $LiNi_{0.8}Co_{0.15}Al_{0.05}O_2$ | Compound 1: 1% | 77.5% | 34.4% |
| Example 14 | $LiNi_{0.8}Co_{0.1}Mn_{0.1}O_2$ | Compound 1: 1% | 71.4% | 36.5% |
| Comparative Example 1 | $LiNi_{0.5}Co_{0.2}Mn_{0.3}O_2$ | - | 63.4% | 57.4% |
| Comparative Example 4 | $LiNi_{1/3}Co_{1/3}Mn_{1/3}O_2$ | - | 54.2% | 50.1% |
| Comparative Example 5 | $LiNi_{1/3}Co_{1/3}Mn_{1/3}O_2$ | Compound 1: 1% | 59.7% | 48.4% |
| Comparative Example 6 | $LiNi_{0.6}Co_{0.2}Mn_{0.2}O_2$ | - | 66.4% | 66.6% |
| Comparative Example 7 | $LiNi_{0.7}Co_{0.1}Mn_{0.2}O_2$ | - | 58.4% | 76.4% |
| Comparative Example 8 | $LiNi_{0.8}Co_{0.15}Al_{0.05}O_2$ | - | 62.2% | 92.2% |
| Comparative Example 9 | $LiNi_{0.8}Co_{0.1}Mn_{0.1}O_2$ | - | 66.6% | 96.4% |

As can be found by comparing Examples 7, 11 to 14 and Comparative Examples 1, 4 to 9, when the nickel content of the positive electrode active material is relatively low, the cycle volume expansion rate in Comparative Example 5 is still 48.4%. The magnitude of improvement between Comparative Example 5 and Comparative Example 4 is much smaller than that between Example 7 and Comparative Example 1, and is also significantly smaller than that between Examples 11 to 14 and Comparative Examples 6 to 9. The cycling performance of the low-nickel battery system in Comparative Example 5 has not been significantly improved, that is, the improvement effect of the compound represented by the formula 1 on the battery performance is significantly related to the nickel content in the positive electrode active material. When the nickel content in the positive electrode active material is higher than a certain value, the compound represented by the formula 1 can play better cooperating role.

3. The test results obtained in Examples 15 to 24 and Comparative Example 6 are shown in Table 3.

Table 3

| Group | Positive electrode material | Compound represented by formula 1 and its mass content | 1000 cycles at 45°C at 1C | |
|---|---|---|---|---|
| | | | Capacity retention rate | Volume expansion rate |
| Example 15 | $LiNi_{0.6}Co_{0.2}Mn_{0.2}O_2$ | Compound 2: 1% | 78.2% | 16.2% |

(continued)

| Group | Positive electrode material | Compound represented by formula 1 and its mass content | 1000 cycles at 45°C at 1C | |
|---|---|---|---|---|
| | | | Capacity retention rate | Volume expansion rate |
| Example 16 | $LiNi_{0.6}Co_{0.2}Mn_{0.2}O_2$ | Compound 6: 1% | 78.1% | 11.5% |
| Example 17 | $LiNi_{0.6}Co_{0.2}Mn_{0.2}O_2$ | Compound 8: 1% | 76.4% | 18.1% |
| Example 18 | $LiNi_{0.6}Co_{0.2}Mn_{0.2}O_2$ | Compound 11: 1% | 77.7% | 17.6% |
| Example 19 | $LiNi_{0.6}Co_{0.2}Mn_{0.2}O_2$ | Compound 15: 1% | 73.2% | 15.4% |
| Example 20 | $LiNi_{0.6}Co_{0.2}Mn_{0.2}O_2$ | Compound 21: 1% | 75.4% | 21.4% |
| Example 21 | $LiNi_{0.6}Co_{0.2}Mn_{0.2}O_2$ | Compound 25: 1% | 78.0% | 14.9% |
| Example 22 | $LiNi_{0.6}Co_{0.2}Mn_{0.2}O_2$ | Compound 35: 1% | 76.1% | 21.4% |
| Example 23 | $LiNi_{0.6}Co_{0.2}Mn_{0.2}O_2$ | Compound 37: 1% | 74.4% | 16.4% |
| Example 24 | $LiNi_{0.6}Co_{0.2}Mn_{0.2}O_2$ | Compound 40: 1% | 73.7% | 17.4% |
| Comparative Example 6 | $LiNi_{0.6}Co_{0.2}Mn_{0.2}O_2$ | - | 66.4% | 66.6% |

As can be seen from the test data in Table 3, when the positive electrode active material is $LiNi_{0.6}Co_{0.2}Mn_{0.2}O_2$, by comparing Examples 15 to 24 and Comparative Example 6, adding the compound represent by the formula 1 can significantly reduce the gas production of the battery during the cycling and improve the high-temperature cycling performance of the battery. At the same time, the high-temperature cycling performance of the battery is improved to varying degrees by using different compounds represented by the formula 1.

4. The test results obtained in Examples 25 to 31 and Comparative Example 7 are shown in Table 4.

Table 4

| Group | Positive electrode material | Compound represented by formula 1 and its mass content | 1000 cycles at 45°C at 1C | |
|---|---|---|---|---|
| | | | Capacity retention rate | Volume expansion rate |
| Example 25 | $LiNi_{0.7}Co_{0.1}Mn_{0.2}O_2$ | Compound 2: 1% | 78.1% | 16.5% |
| Example 26 | $LiNi_{0.7}Co_{0.1}Mn_{0.2}O_2$ | Compound 7: 1% | 78.4% | 20.2% |
| Example 27 | $LiNi_{0.7}Co_{0.1}Mn_{0.2}O_2$ | Compound 14: 1% | 77.7% | 26.4% |
| Example 28 | $LiNi_{0.7}Co_{0.1}Mn_{0.2}O_2$ | Compound 20: 1% | 77.0% | 17.3% |
| Example 30 | $LiNi_{0.7}Co_{0.1}Mn_{0.2}O_2$ | Compound 28: 1% | 76.1% | 18.2% |
| Example 31 | $LiNi_{0.7}Co_{0.1}Mn_{0.2}O_2$ | Compound 35: 1% | 73.4% | 24.9% |
| Comparative Example 7 | $LiNi_{0.7}Co_{0.1}Mn_{0.2}O_2$ | - | 58.4% | 76.4% |

As can be seen from the test data in Table 4, when the positive electrode active material is $LiNi_{0.7}Co_{0.1}Mn_{0.2}O_2$, by comparing Examples 25 to 30 and Comparative Example 7, adding the compound represented by the formula 1 can significantly reduce the gas production of the battery during the cycling and improve the high-temperature cycling performance of the battery. At the same time, the high-temperature cycling performance of the battery is improved to varying degrees by using different compounds represented by the formula 1.

5. The test results obtained in Examples 32 to 35 and Comparative Example 8 are shown in Table 5.

Table 5

| Group | Positive electrode material | Compound represented by formula 1 and its mass content | 1000 cycles at 45°C at 1C | |
|---|---|---|---|---|
| | | | Capacity retention rate | Volume expansion rate |
| Example 32 | $LiNi_{0.8}Co_{0.15}Al_{0.05}O_2$ | Compound 1: 1% | 74.4% | 34.4% |
| Example 33 | $LiNi_{0.8}Co_{0.15}Al_{0.05}O_2$ | Compound 5: 1% | 78.3% | 27.5% |
| Example 34 | $LiNi_{0.8}Co_{0.15}Al_{0.05}O_2$ | Compound 25: 1% | 77.0% | 28.3% |
| Example 35 | $LiNi_{0.8}Co_{0.15}Al_{0.05}O_2$ | Compound 33: 1% | 78.4% | 25.2% |
| Comparative Example 8 | $LiNi_{0.8}Co_{0.15}Al_{0.05}O_2$ | - | 62.2% | 92.2% |

As can be seen from the test data in Table 5, when the positive electrode active material is $LiNi_{0.8}Co_{0.15}Al_{0.05}O_2$, by comparing Examples 32 to 35 and Comparative Example 8, adding the compound represented by the formula 1 can significantly reduce the gas production of the battery during the cycling and significantly improve the capacity retention rate of the battery during the high-temperature cycling.

6. The test results obtained in Examples 36 to 42 and Comparative Example 9 are shown in Table 6.

Table 6

| Group | Positive electrode material | Compound represented by formula 1 and its mass content | 1000 cycles at 45°C at 1C | |
|---|---|---|---|---|
| | | | Capacity retention rate | Volume expansion rate |
| Example 36 | $LiNi_{0.8}Co_{0.1}Mn_{0.1}O_2$ | Compound 1: 1% | 78.5% | 36.5% |
| Example 37 | $LiNi_{0.8}Co_{0.1}Mn_{0.1}O_2$ | Compound 2: 1% | 77.6% | 36.5% |
| Example 38 | $LiNi_{0.8}Co_{0.1}Mn_{0.1}O_2$ | Compound 9: 1% | 78.8% | 30.2% |
| Example 39 | $LiNi_{0.8}Co_{0.1}Mn_{0.1}O_2$ | Compound 13: 1% | 77.3% | 36.4% |
| Example 40 | $LiNi_{0.8}Co_{0.1}Mn_{0.1}O_2$ | Compound 22: 1% | 78.2% | 27.3% |
| Example 41 | $LiNi_{0.8}Co_{0.1}Mn_{0.1}O_2$ | Compound 30: 1% | 77.7% | 28.2% |
| Example 42 | $LiNi_{0.8}Co_{0.1}Mn_{0.1}O_2$ | Compound 40: 1% | 78.1% | 34.9% |
| Comparative Example 9 | $LiNi_{0.8}Co_{0.1}Mn_{0.1}O_2$ | - | 66.6% | 96.4% |

As can be seen from the test data in Table 6, when the positive electrode active material is $LiNi_{0.8}Co_{0.1}Al_{0.1}O_2$, by comparing Examples 36 to 42 and Comparative Example 9, adding the compound represented by the formula 1 can significantly reduce the gas production of the battery during the cycling and significantly improve the capacity retention rate of the battery during the high-temperature cycling.

7. The lithium-ion batteries obtained in Example 7 and Comparative Example 1 were placed in an oven with a constant temperature of 45°C, charged at a constant current of 1C to 4.4 V ($LiNi_{0.5}Co_{0.2}Mn_{0.3}O_2$/AG), then charged at a constant current and a constant voltage until the current drops to 0.05C, and then discharged to 3.0 V at a constant current of 1C, so as to circulate in this way for 1000 cycles. The lithium-ion batteries were disassembled, and the positive electrodes were taken out for transmission electron microscope observation. The TEM image of the positive electrode in Example 7 is shown in FIG. 1, and the TEM image of the positive electrode in Comparative Example 1 is shown in FIG. 2.

By comparing the transmission electron microscope images of FIG. 1 and FIG. 2, when the electrolyte does not contain the compound represented by the formula 1, it is observed that the lattice fringes of the positive electrode material after cycling in Comparative Example 1 are obviously disordered, that is, the structure of the positive electrode material has changed. In combination with the battery performance data in Table 1, it can be known that the volume expansion rate after cycling in Comparative Example 1 is 57.4%. However, the lattice stripes of the positive electrode material in Example 7 in FIG. 2 are still orderly distributed, and the volume expansion rate after cycling in Example 7 is

only 7.2%, indicating that adding the additive represented by the formula 1 can indeed protect the positive electrode material and inhibit the gas production during the cycling.

The above descriptions are only preferred embodiments of the present disclosure, and are not intended to limit the present disclosure. Any modifications, equivalent replacements and improvements made within the range of spirit and principles of the present disclosure should be included in the protection scope of the present disclosure.

## Claims

1. A lithium-ion battery, comprising a positive electrode, a negative electrode and a non-aqueous electrolyte, wherein the positive electrode comprises a positive electrode material layer, the positive electrode material layer comprises a positive electrode active material, and the positive electrode active material comprises $LiNi_xCo_yMn_zL_{(1-x-y-z)}O_2$, where L is Al, Sr, Mg, Ti, Ca, Zr, Zn, Si, Cu, V or Fe, $0.5 \le x \le 1$, $0 \le y \le 0.5$, $0 \le z \le 0.5$, $0 \le x + y + z \le 1$, and an upper limit voltage of the lithium-ion battery is $\ge 4.2$ V;

   wherein the non-aqueous electrolyte comprises a solvent, an electrolyte salt and a compound represented by formula 1:

   A-D-B-E-C Formula 1

   where A, B, and C are each independently selected from a group containing a cyclic carbonate group, a cyclic sulfate group, a cyclic sulfite group, a cyclic sulfonate group, a cyclic sulfone group, a cyclic sulfoxide group, a cyclic carboxylate group or a cyclic anhydride group;
   D and E are each independently selected from a single bond, or a group containing a hydrocarbylene group, an ether bond, a sulfur-oxygen double bond or a carbon-oxygen double bond;
   wherein based on a total mass of the non-aqueous electrolyte as 100%, the compound represented by the formula 1 is added in an amount of 0.01 to 5.0%.

2. The lithium-ion battery according to claim 1, wherein A, B, and C each independently contain 1 to 5 of the cyclic carbonate group(s), the cyclic sulfate group(s), the cyclic sulfite group(s), the cyclic sulfonate group(s), the cyclic sulfone group(s), the cyclic sulfoxide group(s), the cyclic carboxylate group(s) or the cyclic anhydride group(s), and a total number of the cyclic carbonate group(s), the cyclic sulfate group(s), the cyclic sulfite group(s), the cyclic sulfonate group(s), the cyclic sulfone group(s), the cyclic sulfoxide group(s), the cyclic carboxylate group(s) or the cyclic anhydride group(s) of A, B, and C is less than or equal to 10.

3. The lithium-ion battery according to claim 1, wherein A and C are each independently selected from a group represented by formula 2:

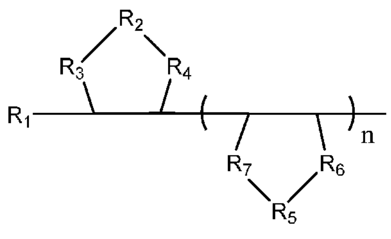

Formula 2

where n is selected from an integer of 0 to 4, and $R_1$ is selected from hydrogen, halogen, a C1 to C5 hydrocarbonyl group or a halohydrocarbonyl group; $R_2$, $R_3$, $R_4$, $R_5$, $R_6$, and $R_7$ are each independently selected from a C1 to C3 hydrocarbylene group, a C1 to C3 alkoxy group, an oxygen atom,

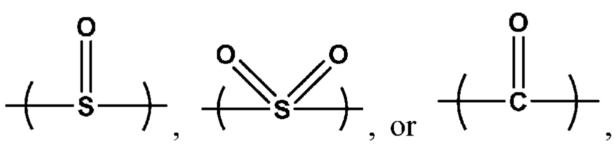

wherein at least one of $R_2$, $R_3$, and $R_4$ is selected from

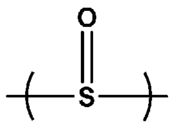, 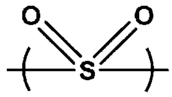, or 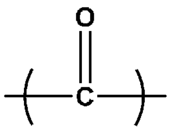,

and at least one of $R_2$, $R_3$, and $R_4$ is selected from the oxygen atom, and at least one of $R_5$, $R_6$, and $R_7$ is selected from

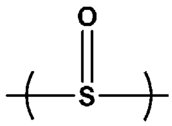,

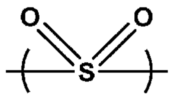, or 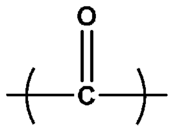,

and at least one of $R_5$, $R_6$, and $R_7$ is selected from the oxygen atom.

**4.** The lithium-ion battery according to claim 1, wherein B is selected from a group represented by formula 3:

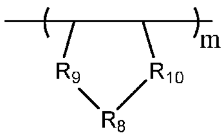

Formula 3

where m is selected from an integer of 1 to 4, and $R_8$, $R_9$, and $R_{10}$ are each independently selected from a C1 to C3 hydrocarbylene group, a C1 to C3 alkoxy group, an oxygen atom,

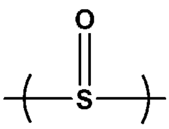, 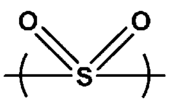, or 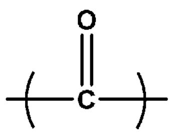,

at least one of $R_8$, $R_9$, and $R_{10}$ is selected from

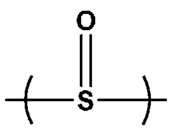, 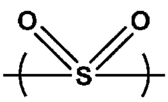, or 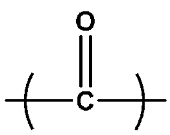,

and at least one of $R_8$, $R_9$, and $R_{10}$ is selected from the oxygen atom.

**5.** The lithium-ion battery according to claim 1, wherein D and E are each independently selected from a group represented by formula 4:

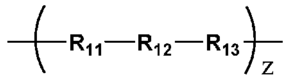

Formula 4

where z is selected from an integer of 0 to 4, $R_{11}$ and $R_{13}$ are each independently selected from a single bond or a C1 to C5 hydrocarbylene group, and $R_{12}$ is selected from a single bond,

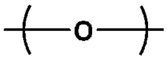, 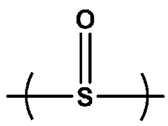, 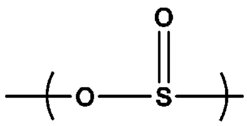, 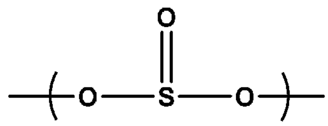, 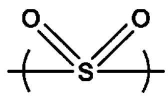,

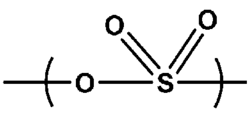, 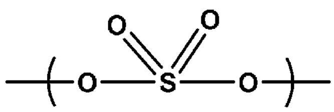, 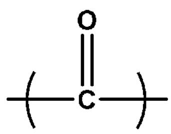, 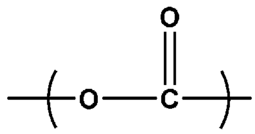

or

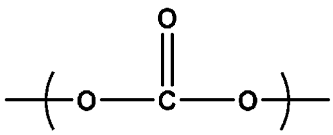.

6. The lithium-ion battery according to claim 1, wherein D and E are each independently selected from the single bond or the C1 to C5 hydrocarbylene group, and A, B, and C are each independently selected from the cyclic carbonate group, the cyclic sulfate group, the cyclic sulfite group, the cyclic sulfonate group, the cyclic sulfone group, the cyclic sulfoxide group, the cyclic carboxylate group or the cyclic anhydride group, which is substituted or unsubstituted; preferably, when A, B or C is substituted, a substituent is selected from halogen, a hydrocarbonyl group or a halohydrocarbonyl group, more preferably, when A, B or C is substituted, the substituent is selected from halogen, an alkyl group or a haloalkyl group.

7. The lithium-ion battery according to any one of claims 1 to 6, wherein
A and C are same as each other, A and B are same as or different from each other, and D and E are same as each other.

8. The lithium-ion battery according to claim 1, wherein the compound represented by the formula 1 is selected from one or more of following compounds:

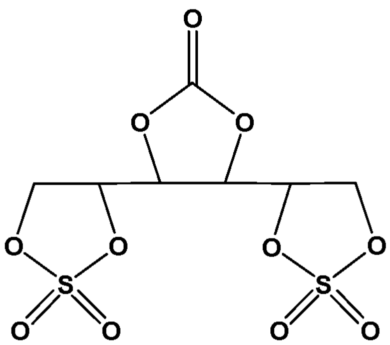

Compound 1

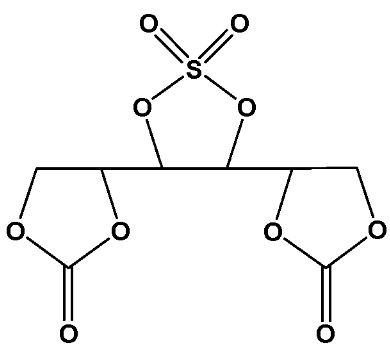

Compound 2

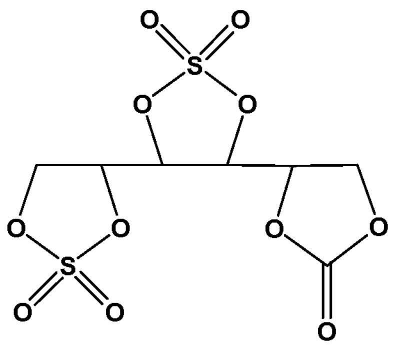

Compound 3

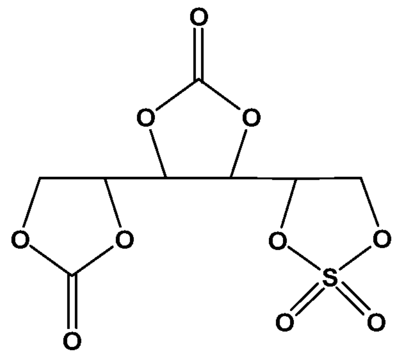

Compound 4

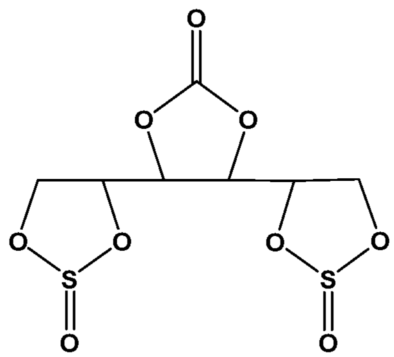

Compound 5

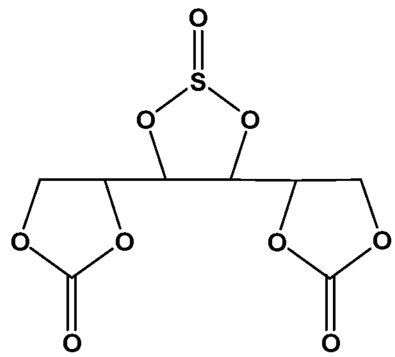

Compound 6

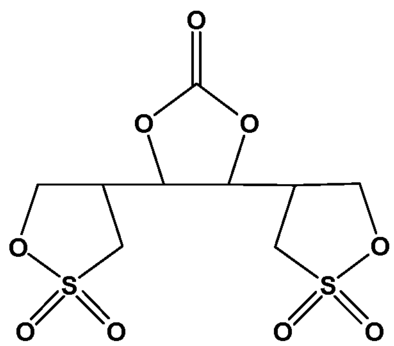

Compound 7

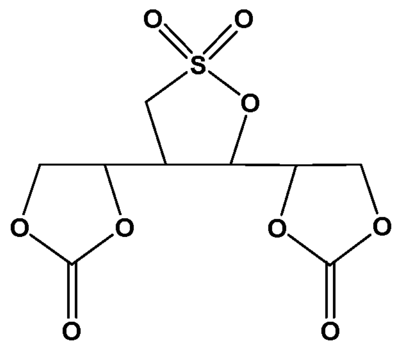

Compound 8

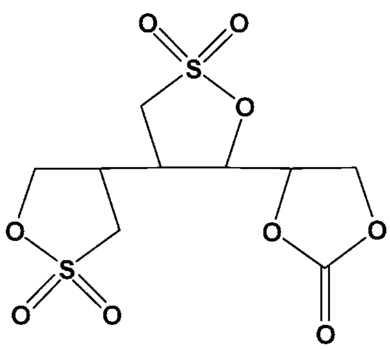

Compound 9

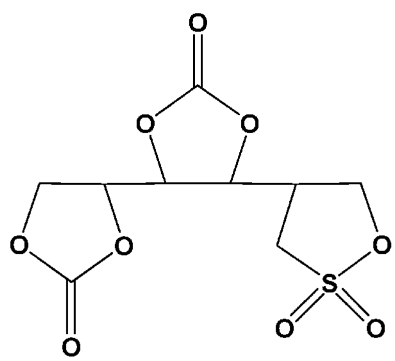

Compound 10

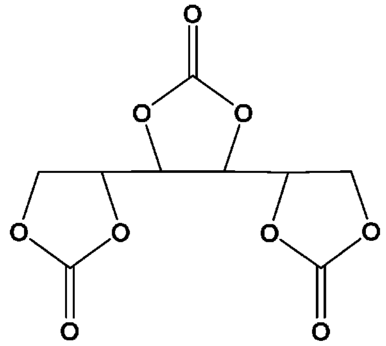

Compound 11

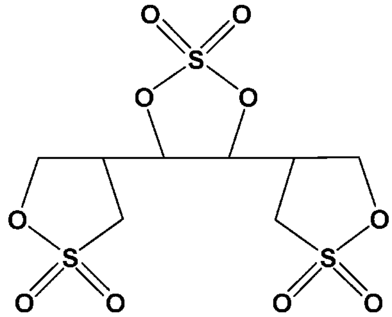

Compound 12

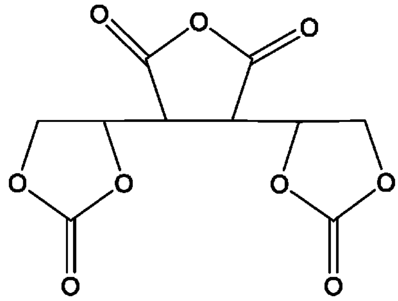

Compound 1-13

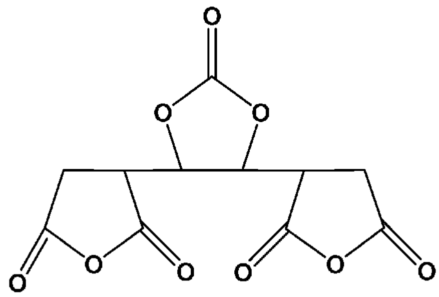

Compound 1-14

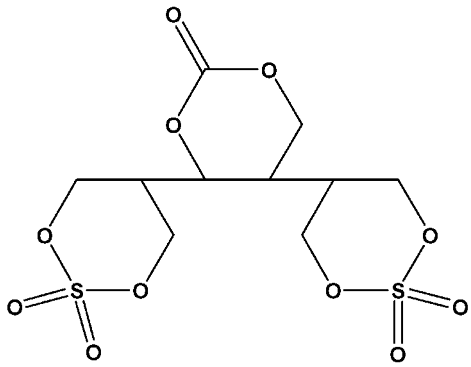

Compound 1-15

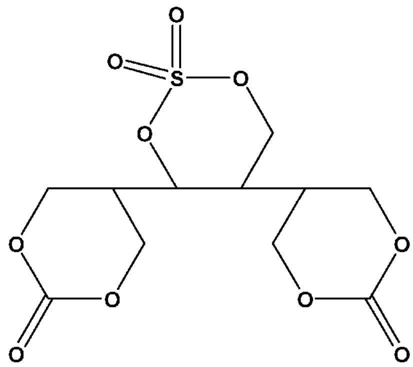

Compound 1-16

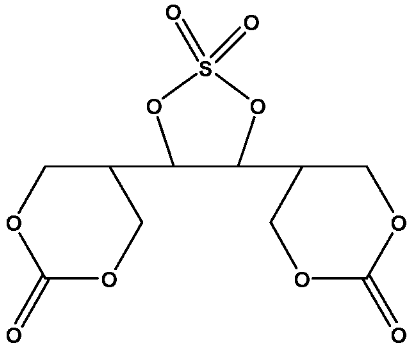

Compound 17

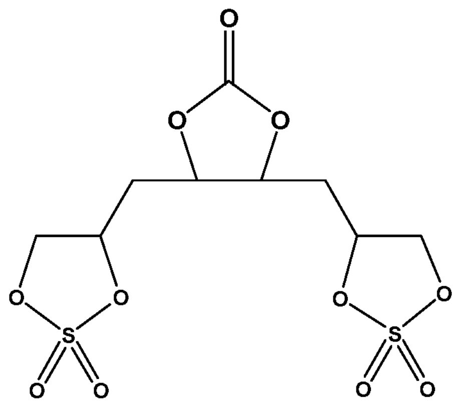

Compound 18

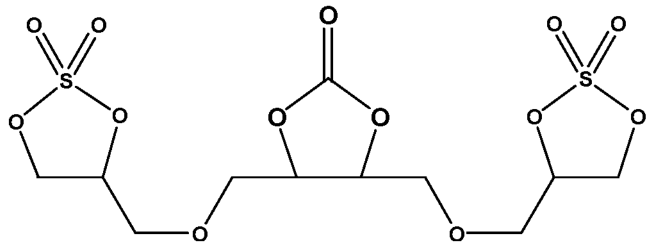

Compound 19

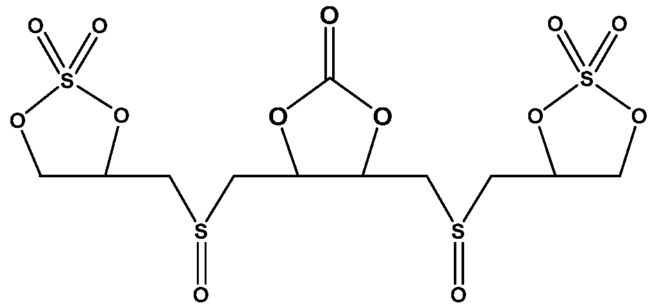

Compound 20

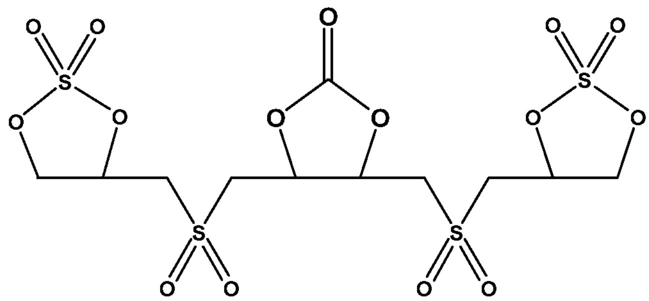

Compound 21

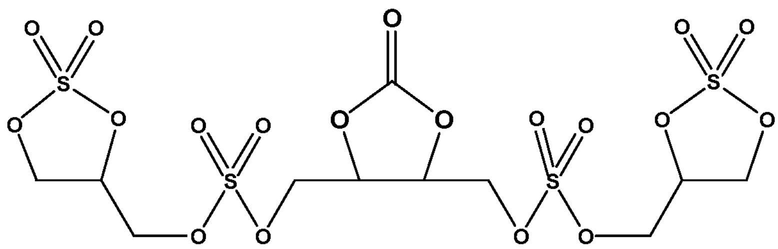

Compound 22

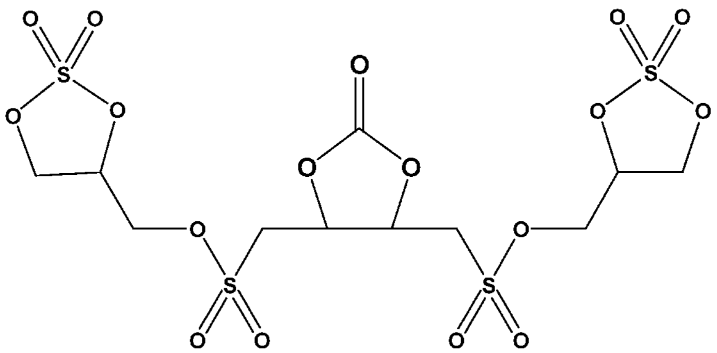

Compound 23

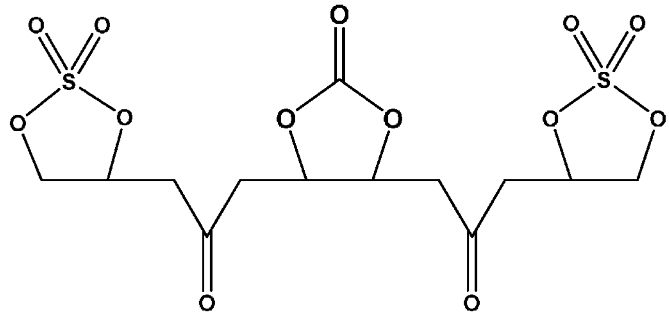

Compound 24

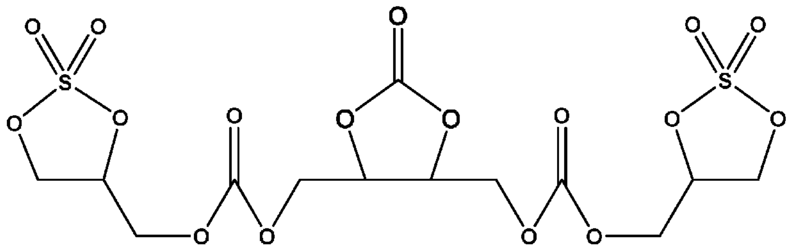

Compound 25

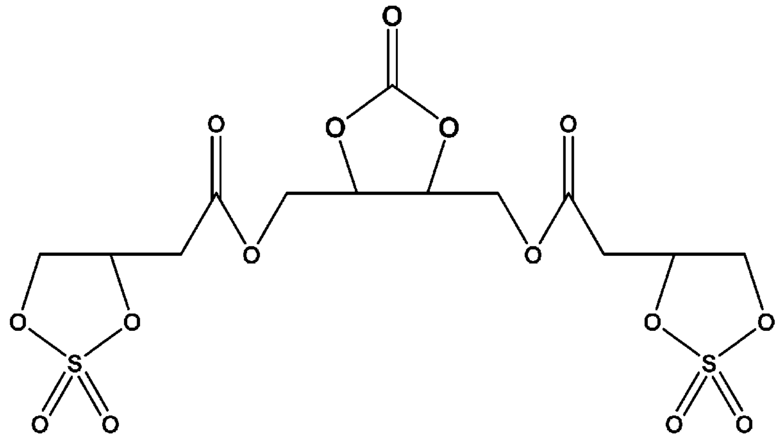

Compound 26

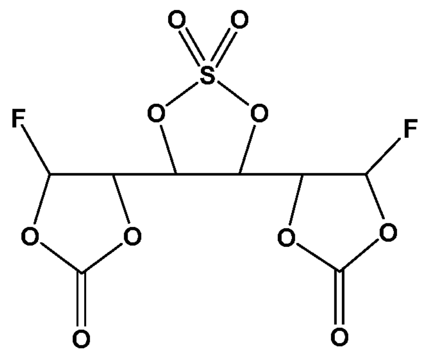

Compound 27

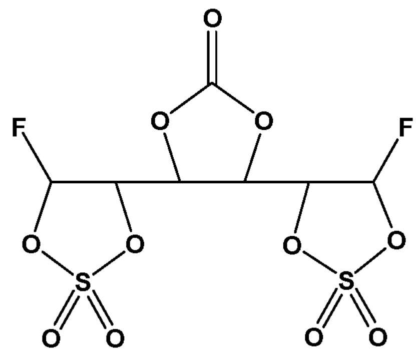

Compound 28

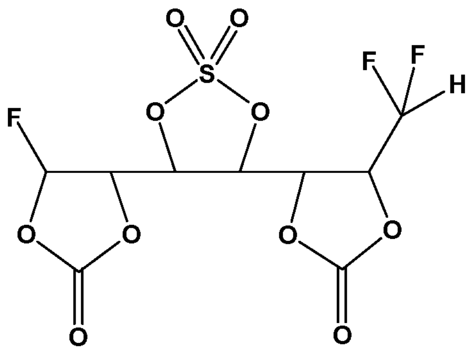

Compound 29

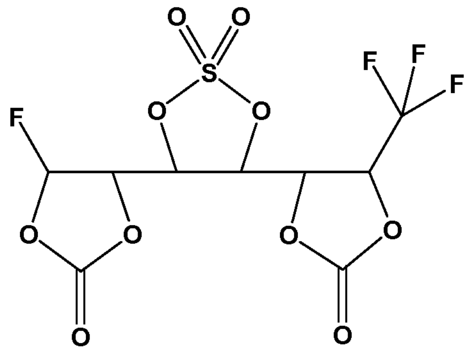

Compound 30

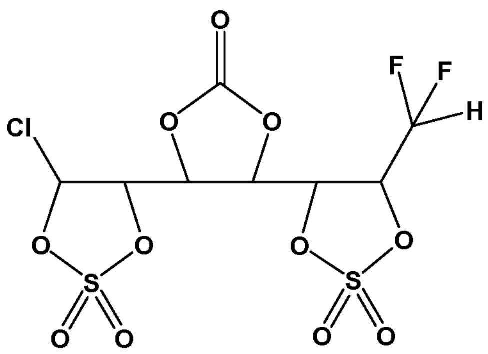

Compound 31

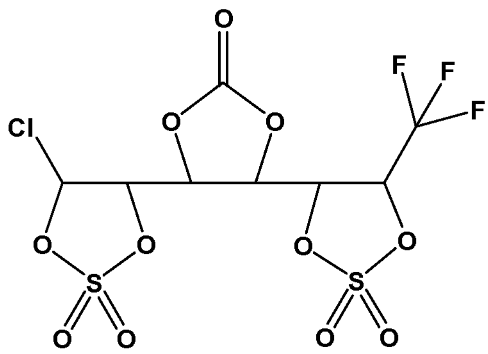

Compound 32

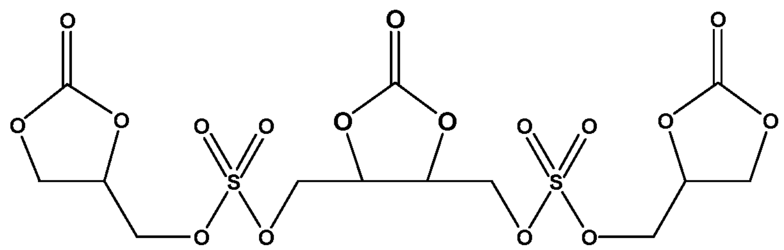

Compound 33

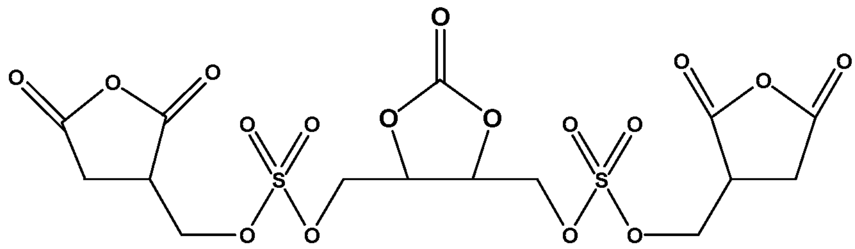

Compound 34

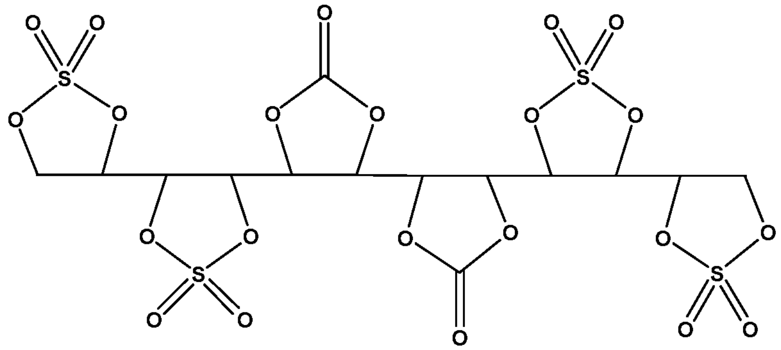

Compound 35

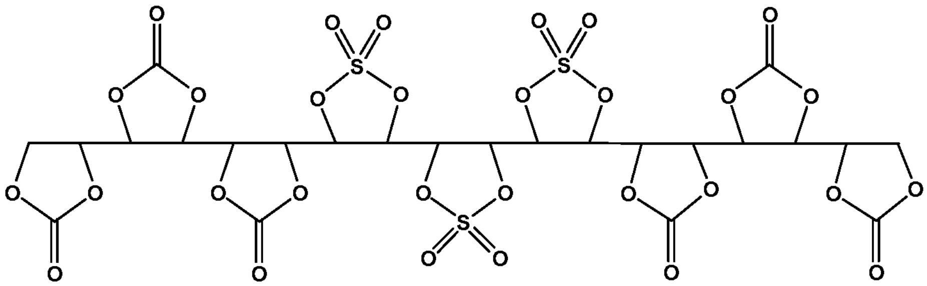

Compound 36

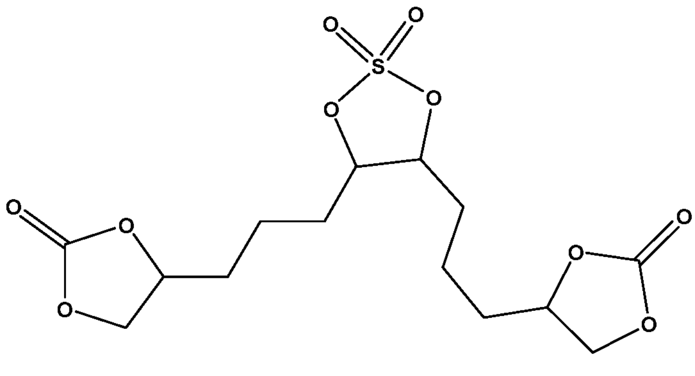

Compound 37

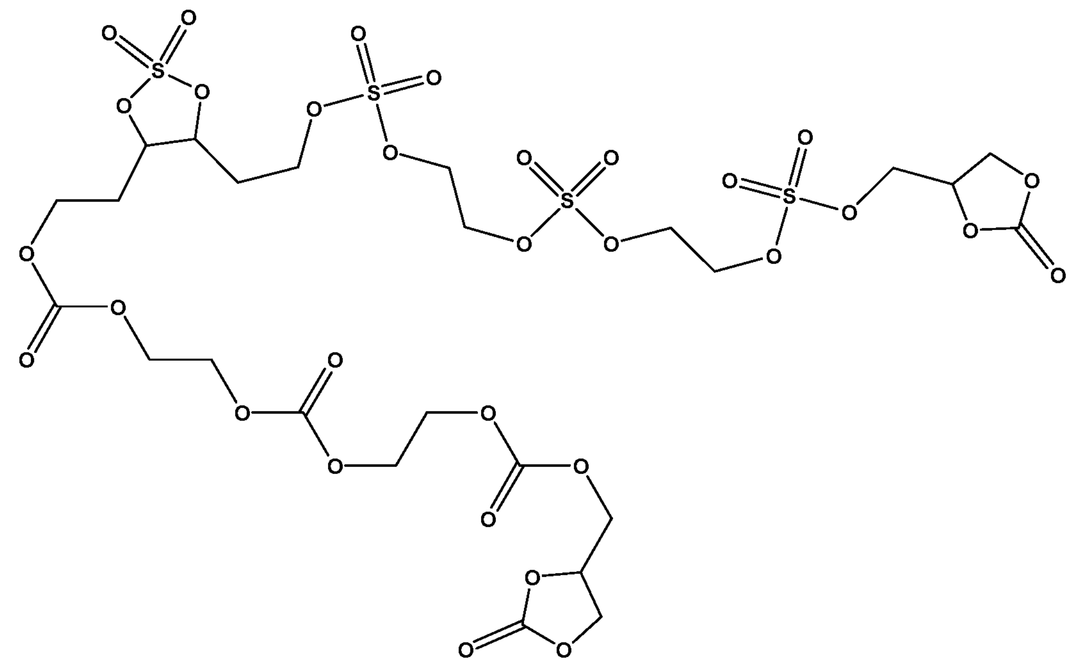

Compound 38

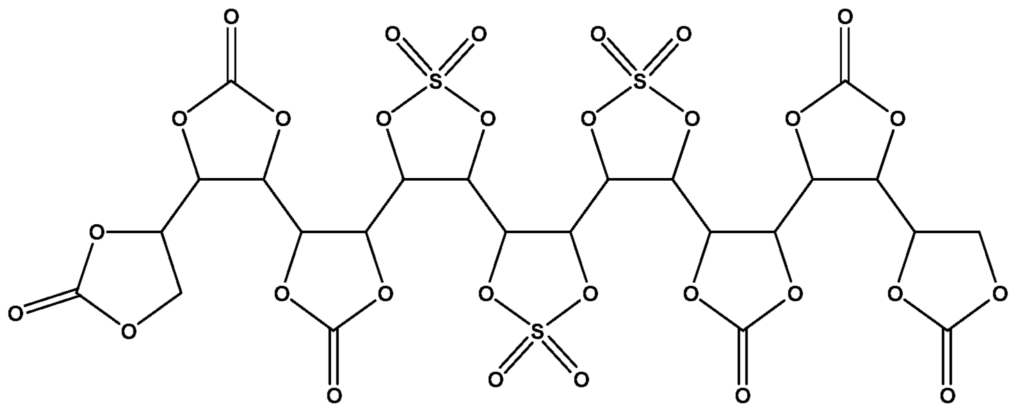

Compound 39

and

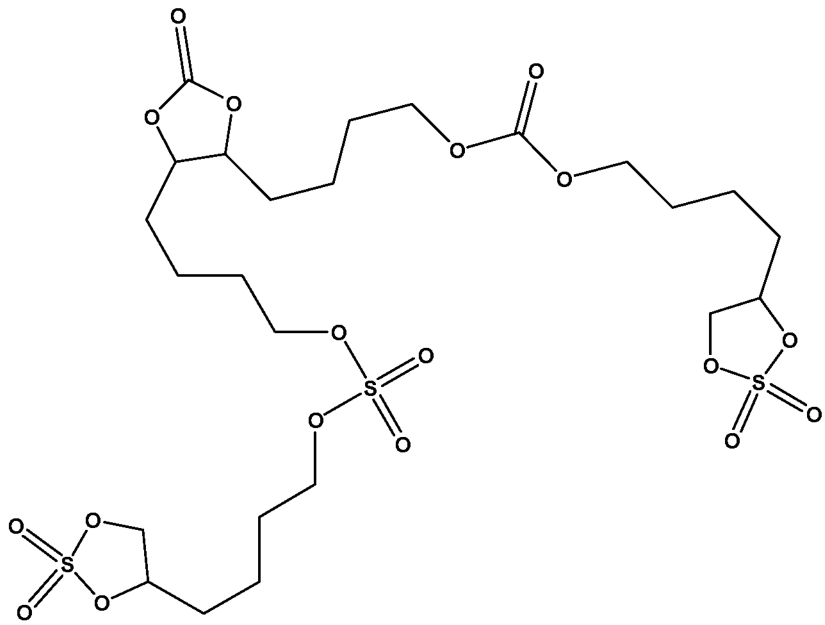

Compound 40.

9. The lithium-ion battery according to claim 1, wherein the positive electrode active material is selected from one or more of $LiNi_{0.5}Co_{0.2}Mn_{0.3}O_2$, $LiNi_{0.6}Co_{0.2}Mn_{0.2}O_2$, $LiNi_{0.7}Co_{0.1}Mn_{0.2}O_2$, $LiNi_{0.8}Co_{0.15}Al_{0.05}O_2$, $LiNi_{0.8}Co_{0.1}Mn_{0.1}O_2$, $LiNi_{0.6}Mn_{0.4}O_2$, and $LiNi_{0.8}Mn_{0.2}O_2$.

10. The lithium-ion battery according to claim 1, wherein the non-aqueous electrolyte further comprises an auxiliary additive comprising at least one of an unsaturated cyclic carbonate compound, a fluorinated cyclic carbonate compound, an aromatic additive, a fluorine-containing anisole compound, a dicarboxylic acid anhydride, lithium difluorophosphate, and lithium bisfluorosulfonimide (LiFSI).

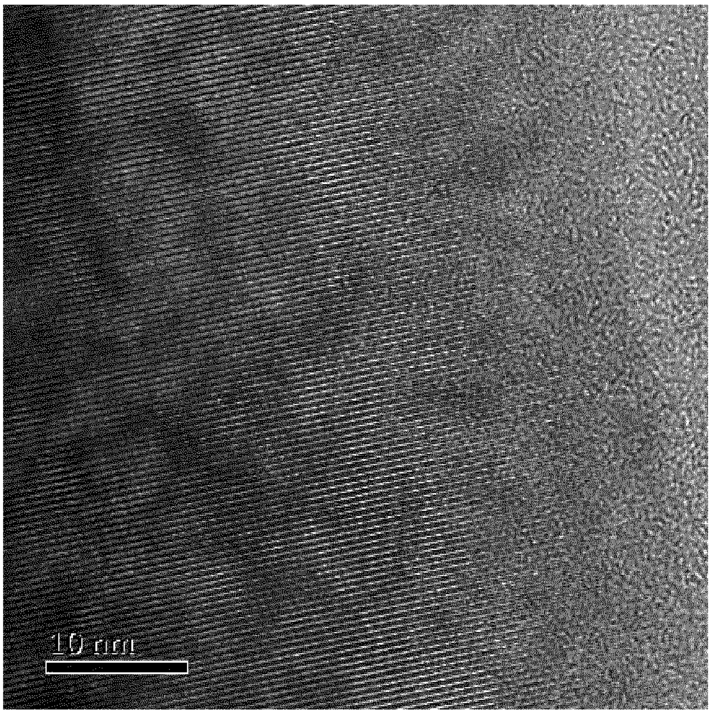


FIG. 1

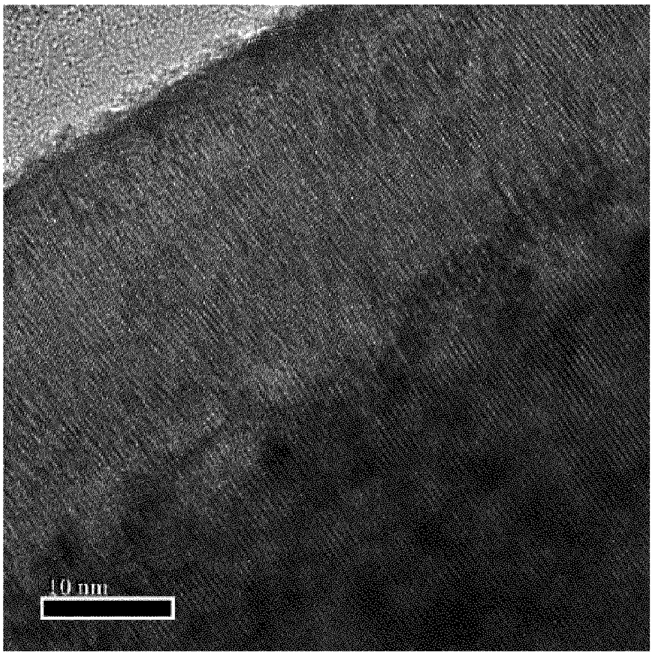


FIG. 2

## INTERNATIONAL SEARCH REPORT

International application No.
**PCT/CN2021/138677**

**A. CLASSIFICATION OF SUBJECT MATTER**

H01M 10/0525(2010.01)i; H01M 10/0567(2010.01)i; H01M 10/0566(2010.01)i; H01M 4/485(2010.01)i; H01M 4/505(2010.01)i; H01M 4/525(2010.01)i

According to International Patent Classification (IPC) or to both national classification and IPC

**B. FIELDS SEARCHED**

Minimum documentation searched (classification system followed by classification symbols)

H01M

Documentation searched other than minimum documentation to the extent that such documents are included in the fields searched

Electronic data base consulted during the international search (name of data base and, where practicable, search terms used)

CNABS; CNTXT; CNKI; SIPOABS; DWPI; USTXT; WOTXT; EPTXT; ISI: 新宙邦, 电解液, 添加剂, 环, 碳酸酯, 硫酸酯, 磺酸酯, 砜, 羧酸酯, 酸酐, 镍基, 高镍, 镍酸锂, 三元, electrolyte, additive, cyclic, carbonate, sulfate, sulfite, carboxylic, anhydride, sulfone, sulfoxide, sulfonate

**C. DOCUMENTS CONSIDERED TO BE RELEVANT**

| Category* | Citation of document, with indication, where appropriate, of the relevant passages | Relevant to claim No. |
|---|---|---|
| X | CN 109428120 A (SHENZHEN CAPCHEM TECHNOLOGY CO., LTD.) 05 March 2019 (2019-03-05)<br>description paragraphs [0024]-[0080] | 1, 3-7, 9, 10 |
| A | CN 109428120 A (SHENZHEN CAPCHEM TECHNOLOGY CO., LTD.) 05 March 2019 (2019-03-05)<br>description paragraphs [0024]-[0080] | 2, 8 |
| X | CN 108258311 A (SHENZHEN CAPCHEM TECHNOLOGY CO., LTD.) 06 July 2018 (2018-07-06)<br>description, paragraphs [0025]-[0047] | 1, 3-7, 9, 10 |
| A | CN 108258311 A (SHENZHEN CAPCHEM TECHNOLOGY CO., LTD.) 06 July 2018 (2018-07-06)<br>description, paragraphs [0025]-[0047] | 2, 8 |
| A | CN 110931863 A (SHENZHEN BAK BATTERY CO., LTD.) 27 March 2020 (2020-03-27)<br>entire document | 1-10 |
| A | CN 103098290 A (MITSUI CHEMICALS, INC.) 08 May 2013 (2013-05-08)<br>entire document | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 February 2022** | **04 March 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/138677**

**C. DOCUMENTS CONSIDERED TO BE RELEVANT**

| Category* | Citation of document, with indication, where appropriate, of the relevant passages | Relevant to claim No. |
|---|---|---|
| A | CN 110100336 A (MURATA MANUFACTURING CO., LTD. et al.) 06 August 2019 (2019-08-06)<br>entire document | 1-10 |
| A | WO 2013038842 A1 (NEC CORP. et al.) 21 March 2013 (2013-03-21)<br>entire document | 1-10 |

Form PCT/ISA/210 (second sheet) (January 2015)

**INTERNATIONAL SEARCH REPORT**
**Information on patent family members**

International application No.
**PCT/CN2021/138677**

| Patent document cited in search report | | | Publication date (day/month/year) | Patent family member(s) | | | Publication date (day/month/year) |
|---|---|---|---|---|---|---|---|
| CN | 109428120 | A | 05 March 2019 | EP | 3678248 | A1 | 08 July 2020 |
| | | | | EP | 3678248 | A4 | 08 July 2020 |
| | | | | WO | 2019041696 | A1 | 07 March 2019 |
| | | | | US | 2020227784 | A1 | 16 July 2020 |
| | | | | CN | 109428120 | B | 17 September 2021 |
| CN | 108258311 | A | 06 July 2018 | CN | 108258311 | B | 10 July 2020 |
| CN | 110931863 | A | 27 March 2020 | WO | 2021093296 | A1 | 20 May 2021 |
| CN | 103098290 | A | 08 May 2013 | KR | 20130043221 | A | 29 April 2013 |
| | | | | KR | 101449353 | B1 | 08 October 2014 |
| | | | | WO | 2012053644 | A1 | 26 April 2012 |
| | | | | CN | 103098290 | B | 13 May 2015 |
| | | | | US | 2013171514 | A1 | 04 July 2013 |
| | | | | US | 9227950 | B2 | 05 January 2016 |
| | | | | JP | 5524347 | B2 | 18 June 2014 |
| | | | | EP | 2631981 | A1 | 28 August 2013 |
| | | | | EP | 2631981 | A4 | 10 September 2014 |
| | | | | EP | 2631981 | B1 | 17 August 2016 |
| | | | | IN | 329323 | B | 17 January 2020 |
| | | | | IN | 201302012 | P1 | 31 October 2014 |
| CN | 110100336 | A | 06 August 2019 | JP | 2020502739 | A | 23 January 2020 |
| | | | | JP | 6934941 | B2 | 15 September 2021 |
| | | | | WO | 2018116529 | A1 | 28 June 2018 |
| | | | | CA | 3128646 | A1 | 28 June 2018 |
| | | | | EP | 3560013 | A1 | 30 October 2019 |
| | | | | CA | 3041783 | A1 | 28 June 2018 |
| | | | | US | 2018183093 | A1 | 28 June 2018 |
| | | | | US | 10497966 | B2 | 03 December 2019 |
| | | | | KR | 20190061066 | A | 04 June 2019 |
| | | | | KR | 102247969 | B1 | 03 May 2021 |
| | | | | IN | 201917023297 | A | 02 August 2019 |
| WO | 2013038842 | A1 | 21 March 2013 | JP | 6032204 | B2 | 24 November 2016 |

Form PCT/ISA/210 (patent family annex) (January 2015)